(19) Europäisches Patentamt
European Patent Office
Office européen des brevets

(11) EP 2 358 861 B1

# (12) FASCICULE DE BREVET EUROPEEN

(45) Date de publication et mention de la délivrance du brevet:
**08.02.2017 Bulletin 2017/06**

(21) Numéro de dépôt: **09803793.0**

(22) Date de dépôt: **18.12.2009**

(51) Int Cl.:
***C12N 1/16*** (2006.01) ***C12P 7/64*** (2006.01)
***C12Q 3/00*** (2006.01) ***G05D 21/00*** (2006.01)

(86) Numéro de dépôt international:
**PCT/FR2009/001465**

(87) Numéro de publication internationale:
**WO 2010/076432 (08.07.2010 Gazette 2010/27)**

## (54) NOUVEAU PROCÉDÉ DE CULTURE DE LEVURES DU GENRE YARROWIA

NEUES VERFAHREN ZUR KULTIVIERUNG VON YARROWIA-HEFEN

NOVEL METHOD FOR CULTIVATING YEASTS OF THE YARROWIA GENUS

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **19.12.2008 FR 0807252**

(43) Date de publication de la demande:
**24.08.2011 Bulletin 2011/34**

(73) Titulaires:
- **Airbus Operations**
  **31060 Toulouse (FR)**
- **Centre National de la Recherche Scientifique (C.N.R.S.)**
  **75794 Paris Cedex 16 (FR)**

(72) Inventeurs:
- **COSTES, Philippe**
  **F-31000 Toulouse (FR)**
- **URIBELARREA, Jean-Louis**
  **F-31400 Toulouse (FR)**
- **CESCUT, Julien**
  **F-11410 Saint Michel de Lanes (FR)**
- **MOLINA-JOUVE, Carole**
  **F-34800 Toulouse (FR)**

(74) Mandataire: **Santarelli**
**146, rue Paradis**
**13294 Marseille Cedex 6 (FR)**

(56) Documents cités:
**WO-A2-2008/000277 US-A1- 2005 136 519**

- **AGGELIS GEORGE ET AL: "Enhancement of single cell oil production by Yarrowia lipolytica growing in the presence of Teucrium polium L. aqueous extract" BIOTECHNOLOGY LETTERS, vol. 21, no. 9, septembre 1999 (1999-09), pages 747-749, XP002537160 ISSN: 0141-5492**
- **PAPANIKOLAOU SERAPHIM ET AL: "Lipid production by Yarrowia lipolytica growing on industrial glycerol in a single-stage continuous culture" BIORESOURCE TECHNOLOGY, vol. 82, no. 1, mars 2002 (2002-03), pages 43-49, XP002537161 ISSN: 0960-8524**
- **CESCUT J.: "Accumulation d'acylglycérols par des espèces levuriennes à usage carburant aéronautique: physiologie et performances de procédés" Thèse en vue de l'obtention du Doctorat de l'Université de Toulouse, [Online] 31 mars 2009 (2009-03-31), XP002579127 L'INSA de Toulouse Extrait de l'Internet: URL:http://eprint.insa-toulouse.fr/archive /00000289/01/Cescut.pdf>**
- **GOTTVALDOVA M ET AL: "ENHANCEMENT OF CELLULASE PRODUCTION BY TRICHODERMA-VIRIDE USING CARBON NITROGEN DOUBLE FED BATCH" BIOTECHNOLOGY LETTERS, vol. 4, no. 4, 1982, pages 229-232, XP002537162 ISSN: 0141-5492**
- **LEE JEONGSEOK ET AL: "Control of fed-batch fermentations" BIOTECHNOLOGY ADVANCES, vol. 17, no. 1, avril 1999 (1999-04), pages 29-48, XP4174768 ISSN: 0734-9750**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- WYNN JAMES P ET AL: "The role of malic enzyme in the regulation of lipid accumulation in filamentous fungi" MICROBIOLOGY (READING), vol. 145, no. 8, août 1999 (1999-08), pages 1911-1917, XP002537164 ISSN: 1350-0872
- JIN M ET AL: "Metabolic flux analysis on arachidonic acid fermentation" FRONTIERS OF CHEMICAL ENGINEERING IN CHINA, vol. 1, no. 4, 2007, pages 421-426, XP002537165
- TSUNEO YAMANÉ ET AL: "Fed-batch techniques in Microbial processes" ADVANCES IN BIOCHEMICAL ENGINEERING/BIOTECHNOLOGY, vol. 30, 1984, pages 147-194, XP8108291 ISSN: 0724-6145
- Mandal Chaitali ET AL: "Productivity Improvement in Xanthan Gum Fermentation Using Multiple Substrate Optimization", BIOTECHNOLOGY PROGRESS., vol. 19, no. 4, 1 January 2003 (2003-01-01), pages 1190-1198, XP055258743, US ISSN: 8756-7938, DOI: 10.1021/bp0257269
- G.A. Montague ET AL: "Fermentation Monitoring and Control: A Perspective", BIOTECHNOLOGY AND GENETIC ENGINEERING REVIEWS, vol. 7, no. 1, 1 December 1989 (1989-12-01), pages 147-188, XP055258770, GB ISSN: 0264-8725, DOI: 10.1080/02648725.1989.10647858

## Description

L'engouement croissant pour les énergies de substitution aux carburants issus du raffinage du pétrole a entraîné de nombreuses recherches dans le domaine.

Plusieurs voies sont investiguées telles que la production microbienne d'huiles, particulièrement d'acides gras polyinsaturés, avec des microorganismes oléagineux capables de convertir des substrats tels que des matières grasses ou du glycérol, en triglycérides et acides gras

Parmi les applications industrielles potentielles de ces processus on distingue particulièrement l'accumulation dans des microorganismes de lipides enrichis en acides gras essentiels, lesdits lipides étant particulièrement destinés à être utilisés comme complément nutritionnel, ou pour la production de combustible sous la forme d'énergie renouvelable en alternative ou en additif aux carburants issus du pétrole.

Du fait de l'importance économique grandissante des sources d'énergie renouvelables, on constate un intérêt grandissant pour l'amplification de l'accumulation des lipides microbiens et la modulation de la composition en fonction des domaines d'application ciblés.

A cet égard les levures du genre *Yarrowia*, particulièrement les levures oléagineuses *Yarrowia lipolytica, Yarrowia sp. KS1, Yarrowia sp. SM15S06, Yarrowia sp. SM-22, Yarrowia sp. SSJO2005, Yarrowia sp. TFM01* présentent un intérêt majeur.

Parmi ces souches de levures, *Yarrowia lipolytica,* est une des levures "non conventionnelles" les plus largement étudiées, en raison de sa capacité d'accumuler une quantité importante de lipides (environ 35% de sa masse sèche) selon un profil spécifique.

Ces levures, particulièrement *Yarrowia lipolytica,* sont capables d'utiliser efficacement des substrats hydrophobes, par exemple des alcanes, des acides gras et des huiles, comme seule source de carbone.

Les chaînes aliphatiques consommées peuvent être utilisées comme substrat de la production d'énergie, de l'anabolisme ou accumulées sous des formes inchangées ou modifiées.

Les molécules de stockage telles que les triglycérides (TG) et/ou les esters de stérols, (sterylesters ; SE), incapables de s'intégrer dans les bicouches de phospholipides, se groupent pour former le noyau hydrophobe desdits corps lipidiques (lipid bodies ; LB) selon la théorie des bourgeonnements des corps lipidiques de Zweytick D..

Lesdits corps lipidiques ont longtemps été uniquement considérés comme un stockage de lipides neutres pouvant être mobilisés en période de privation.

Toutefois, l'hypothèse desdits corps lipidiques comme simple compartiment passif de stockage a dû être modifiée depuis que de nombreuses protéines desdits corps lipidiques ont été identifiées comme des enzymes impliquées dans le métabolisme lipidique, en particulier dans la synthèse et la dégradation des triglycérides.

Si les levures du genre *Yarrowia* et particulièrement la souche *Yarrowia lipolytica* ont été largement étudiées pour leur capacité à convertir différents substrats hydrophobes tels que les acides gras, triacylglycérols, n-alcanes en métabolites extra ou intra-cellulaires de grands intérêt comme par exemple l'acide citrique, la $\gamma$-décalactone, les lipases ou encore les lipides, très peu de travaux ont trait à des cultures de levures du genre *Yarrowia,* et particulièrement de la souche *Yarrowia lipolytica,* avec le glucose comme substrat ou co-substrat provenant de ressources à prix de revient faible et/ou renouvelables.

Il est admis dans l'art antérieur que quand *Yarrowia lipolytica* se développe en condition de limitation d'azote, avec comme seule source de carbone le glucose, elle accumule une quantité de lipides atteignant 0,35 $g_{lipid}.g_x^{-1}$($g_{lipid}$/g de levure).

Cependant, en conditions de limitation d'azote et en présence d'un excès de substrat carboné, il apparaît simultanément à l'accumulation de lipides, une production de quantités remarquables de sous-produits, qui peuvent s'avérer néfastes, en particulier lorsque l'on veut récupérer et/ou transformer les lipides ou encore qui peuvent ralentir voire inhiber la production desdits lipides par la levure.

L'acide citrique est le principal sous-produit qui peut être synthétisé au cours de la phase d'accumulation de lipides, ce qui a comme conséquence de diminuer le rendement de conversion du substrat en lipides.

La production de l'acide citrique est probablement due à un flux de carbone largement excédentaire par rapport au flux d'azote. Dans ce cas on parle d'"over-flow" de flux de carbone dans les voies métaboliques centrales dépassant les capacités anaboliques et cataboliques de la levure.

Des études des flux des principales voies métaboliques dans *Yarrowia lipolytica* ont montré que, théoriquement, la levure est capable de stocker des lipides neutres sans synthèse concomitante d'acide citrique et ce particulièrement dans le cas d'apport contrôlé du substrat. Des études de modélisation des flux métaboliques montrent que l'accumulation intracellulaire de lipides pourrait atteindre, en théorie, jusqu'à 0,035 $g_c.g_x^{-1}.h^{-1}$ (gramme de carbone/gramme de levure/par heure) lors de culture avec un apport contrôlé en substrat.

Ainsi devant ce constat que *Yarrowia lipolytica* pourrait accumuler un taux élevé de lipides en limitation d'azote avec un apport contrôlé de carbone avec une production minimale d'acide citrique, les inventeurs en ont déduit que les conditions idéales de cultures de levures du genre *Yarrowia,* particulièrement de la souche *Yarrowia lipolytica,* pour

obtenir une production maximale de lipides sans production d'acide citrique, nécessitent une gestion élaborée des apports en carbone et azote dans le milieu de culture.

C'est donc un des buts de l'invention que de fournir un procédé de culture de levures du genre *Yarrowia,* particulièrement de la souche *Yarrowia lipolytica,* permettant à la fois l'obtention d'un taux maximum d'accumulation de lipides et de rendement de conversion du substrat en lipides tout en limitant au maximum, voire évitant la production d'acide citrique, par ladite levure.

Ainsi l'invention a pour objet premier un procédé de culture de levures du genre *Yarrowia*, particulièrement de la souche *Yarrowia lipolytica,* caractérisé en ce que, pour favoriser la production de lipides au cours de la culture, le milieu de culture comprend outre les nutriments essentiels nécessaires à la croissance de levures du genre *Yarrowia,* du glucose seul et que la valeur du rapport entre la vitesse de consommation de carbone et la vitesse de consommation d'azote ($r_C/r_N$) est contrôlé et fixé à une valeur définie, très avantageusement lorsque la culture est en phase d'accumulation des lipides.

Comme l'ont démontré les inventeurs (voir par ailleurs dans le texte) il peut être important, pour minimiser la production d'acide citrique au cours de la culture, que simultanément au contrôle du rapport entre la vitesse de consommation de carbone et la vitesse de consommation d'azote ($r_C/r_N$), le rapport entre la vitesse de consommation d'oxygène et la vitesse de consommation d'azote ($r_{O2}/r_N$) soit également maintenu constant. L'équation 7, (voir infra) établit la relation entre le coefficient respiratoire (QR), qui est le rapport entre la vitesse de production de dioxyde de carbone ($r_{co2}$) et la vitesse de consommation d'oxygène ($r_{O2}$), le rapport entre la vitesse de consommation de carbone et la vitesse de consommation d'azote ($r_C/r_N$) et le rapport entre la vitesse de consommation d'oxygène et la vitesse de consommation d'azote ($r_{O2}/r_N$) selon :

$$\frac{\frac{r_C}{r_N}}{QR} = k * \frac{r_{O_2}}{r_N} \quad \text{(Equation 7)}$$

Où k correspond à $\frac{r_C}{r_{CO_2}}$ équivalent à $Y_{S,CO2}$, rendement théorique limite de production de dioxyde de carbone par unité de quantité de substrat consommé, constant pour un métabolisme stabilisé (accumulation de lipides, croissance).

Ainsi selon le procédé selon l'invention, pour optimiser la production de lipides et limiter la production d'acide citrique, on peut contrôler et fixer à des valeurs définies :

- le rapport entre la vitesse de consommation de carbone et la vitesse de consommation de l'azote ($r_C/r_N$) et le coefficient respiratoire (QR) ; ou bien
- le rapport entre la vitesse de consommation de carbone et la vitesse de consommation de l'azote ($r_C/r_N$) et le rapport entre la vitesse de consommation d'oxygène et la vitesse de consommation d'azote ($r_{O2}/r_N$) ou
- le rapport entre la vitesse de consommation d'oxygène et la vitesse de consommation d'azote ($r_{O2}/r_N$) et QR.

Avantageusement selon l'invention, pour optimiser la production de lipides et limiter, voire annihiler, la production d'acide citrique, on pourra contrôler et fixer à des valeurs définies le rapport entre la vitesse de consommation de carbone et la vitesse de consommation de l'azote ($r_C/r_N$) et le rapport ($r_{O2}/r_N$).

Selon l'invention, le rapport entre la vitesse de consommation de carbone et la vitesse de consommation d'azote ($r_C/r_N$) peut être maintenue à une valeur comprise entre 12 et 16, préférentiellement entre 13 et 15 (mole de carbone consommé par mole d'azote consommé).

Selon l'invention encore, la valeur du rapport entre la vitesse de consommation d'oxygène et la vitesse de consommation d'azote ($r_{O2}/r_N$) peut être maintenue entre 21 et 27 préférentiellement entre 22 et 26 (mole d'oxygène consommée par mole de d'azote consommé).

Selon l'invention enfin, la valeur du coefficient respiratoire (QR) peut être maintenue entre 0,9 et 1,30 préférentiellement entre 1,01 et 1,11 (mole de $CO_2$ produite par mole de d'oxygène consommée).

L'Homme du métier comprend que tout procédé de culture de levures du genre *Yarrowia,* particulièrement de la souche *Yarrowia lipolytica,* connu peut être concerné par l'invention pour peu que le rapport ($r_C/r_N$) seul, ou que le rapport ($r_C/r_N$) et le rapport ($r_{O2}/r_N$), ou que le rapport ($r_{O2}/r_N$) et QR ou que le rapport ($r_C/r_N$) et QR soient maintenus dans les domaines de variation des valeurs optimales déterminées par les inventeurs.

Selon un aspect particulier de l'invention, le procédé de culture selon l'invention peut inclure un milieu de culture

comprenant, outre les nutriments essentiels nécessaires à la croissance des levures du genre *Yarrowia,* particulièrement de la souche *Yarrowia lipolytica,* bien connus dans l'art antérieur, du glucose comme seule source de carbone.

Avantageusement selon l'invention, le procédé de culture peut être un procédé par alimentation discontinue (ou Fed-batch).

Cependant, dans l'optique de l'industrialisation de la culture de levures du genre *Yarrowia,* particulièrement de la souche *Yarrowia lipolytica,* pour produire des lipides, il est nécessaire d'automatiser le contrôle du rapport ($r_C/r_N$), particulièrement lorsque les cultures sont des cultures par alimentation discontinue.

L'automatisation des procédés de culture par alimentation discontinue bute sur un manque de moyens de contrôle efficace et complet des variables intervenant au cours de la culture. Des boucles de contrôle efficaces du pH, de la température, de la vitesse d'agitation, de l'anti-mousse et de la densité optique existent sur les fermenteurs récents. Mais à la connaissance des inventeurs peu de travaux sur le contrôle éventuellement basé sur un algorithme de la concentration d'un substrat au cours de la culture et/ou son maintien constant, qui plus est avec une réelle efficacité, existent.

Plusieurs travaux de l'art antérieur mentionnent l'étude des différents modes de contrôle de la concentration en substrat dans les cultures par alimentation discontinue. Les meilleurs résultats ont été obtenus avec un contrôle du substrat de type "feedforward-feedback" basé sur un filtre de Kalman utilisant un système de mesure du glucose en ligne (Arndt, M.et coll., (2005), Computers & Chemical Engineering 29, 1113 ; Hitzmann, B. et coll., (2000), Bioprocess and Biosystems Engineering 23, 337).

Vicente, A. ((1997) Biotechnology and Bioengineering 58, 445-450) a développé une méthode alternative basée sur le contrôle de l'alimentation en glucose couplée à la régulation du pH. En effet, l'estimation de la biomasse en ligne peut être faite par le biais du contrôle du pH dans des systèmes de culture de levures en aérobie. Des études expérimentales montrent l'existence d'une relation directe entre la production de protons et la croissance dans de précédents travaux (Huth, J. et coll., J. Basic Microbiol. 30(7): 481-488, 30(7): 489-497 et 30(8): 561-567 ou encore Roos, W. et coll., (1984). J. Gen. Microbiol. 130: 1007-1014).

De même la détermination de l'équivalent d'acide ou de base consommé par la culture par unité de temps a été largement utilisé comme paramètre pour le contrôle en ligne des procédés de culture (Ishizaki, A. et coll., (1994), J. Ferment. Bioeng 77, 541-547; Iversen, J.J.L. et coll., (1994) Appl. Microbiol. Biotechnol. 42, 256-262 ; San, K.-Y. and Stephanopoulos, G. (1984), Biotechnol. Bioeng 26, 1209-1218 ; Shioya, S. (1989),Elsevier Applied Science 15-22 ; Siano, S.A. (1995), Biotechnol. Bioeng. 47, 651-665).

Il n'en demeure pas moins qu'il demeure un manque de moyens de contrôle efficace et complet des flux de carbone et d'azote ou des flux de carbone et d'azote et des flux d'oxygène et d'azote au cours de la culture, particulièrement des cultures de levures du genre *Yarrowia,* particulièrement de la souche *Yarrowia lipolytica.*

C'est un des autres buts de la présente invention que de fournir un tel moyen.

En effet, les inventeurs ont optimisé, en termes de productivité et de rendement, l'accumulation intracellulaire de lipides par les levures du genre *Yarrowia,* particulièrement de la souche *Yarrowia lipolytica,* en culture, particulièrement en culture par alimentation discontinue, très particulièrement sur milieu de culture comprenant des minéraux et au moins une source du carbone, avantageusement du glucose.

A cet égard les inventeurs ont mis au point un nouveau mode de contrôle du débit de substrat dérivé d'un régulateur PID pour la culture en alimentation contrôlée (Fed-Batch) de levures du genre *Yarrowia,* particulièrement de la souche *Yarrowia lipolytica,* produisant une quantité élevée de lipides, sans production concomitante d'acide citrique.

Un contrôleur PID (pour contrôleur Proportionnel-Intégral-Dérivé) est un terme générique désignant un algorithme permettant le contrôle, au travers d'une boucle fermée de rétrocontrôle, des systèmes industriels automatisés.

Un contrôleur PID vise à corriger l'écart (nommé erreur) entre la valeur mesurée d'une variable d'un procédé et la valeur théorique souhaitée (point de consigne) de la même variable à un point donné, par le calcul et la mise en oeuvre d'une action correctrice qui peut ajuster en conséquence le déroulement du procédé. L'algorithme constituant l'élément calculateur du contrôleur PID comporte trois paramètres, la valeur proportionnelle, la valeur intégrale et la valeur dérivée d'un élément choisi.

Selon l'invention l'élément choisi sera le débit du glucose introduit dans le dispositif de culture dans lequel la culture de levures du genre *Yarrowia,* particulièrement de la souche *Yarrowia lipolytica,* est réalisée.

Ainsi l'invention a aussi pour objet un procédé de culture de levures du genre *Yarrowia,* particulièrement de la souche *Yarrowia lipolytica,* caractérisé en ce que

- le rapport entre la vitesse de consommation de carbone et la vitesse de consommation de l'azote ($r_C/r_N$) et le coefficient respiratoire (QR) ; ou bien
- le rapport entre la vitesse de consommation de carbone et la vitesse de consommation de l'azote ($r_C/r_N$) et le rapport entre la vitesse de consommation d'oxygène et la vitesse de consommation d'azote ($r_{O2}/r_N$) ou
- le rapport entre la vitesse de consommation d'oxygène et la vitesse de consommation d'azote ($r_{O2}/r_N$) et QR ;

dans la culture est maintenu constant par l'action d'un contrôleur de type PID.

Selon un mode de réalisation particulier de l'invention, le contrôleur de type PID pourra intervenir sur le débit du glucose introduit dans le fermenteur dans lequel la culture de levures du genre *Yarrowia,* particulièrement de la souche *Yarrowia lipolytica* est réalisée. Cela peut être réalisé par intervention sur le débit nominal de la pompe d'alimentation en glucose.

Plus précisément au cours de leurs travaux les inventeurs ont utilisé des cultures en alimentation contrôlée (Fed-Batch) pour contrôler l'alimentation en éléments nutritifs (substrats, des sels, des vitamines, des sources d'azote, ...) et pour maîtriser les comportements des levures en conditions optimales d'accumulation qui sont :

- une limitation en azote, qui limite la croissance ;
- un flux de carbone adapté à la croissance et à la néo-synthèse des lipides sans production d'acide citrique.

Afin de mettre au point le contrôleur de type PID, les inventeurs ont utilisé les paramètres suivants :

- quotient respiratoire QR estimé à partir de la mesure de la composition des gaz en entrée et en sortie du fermenteur et des équations de bilan de matière ;
- le rapport $r_C/r_N$ estimé par des mesures en ligne des flux de matière d'entrée et de sortie en carbone et en azote dans le fermenteur ;
- le temps de fonctionnement de la pompe d'alimentation en composé basique (nécessaire pour la neutralisation des acides néoformés).

Le quotient respiratoire (QR) se définit comme le rapport de la vitesse de production de $CO_2$ par la vitesse de consommation d'$O_2$. Cette production et cette consommation peuvent être reliées au métabolisme cellulaire selon l'équation réactionnelle suivante (équation 1)

$$\textbf{Sub.} + \textbf{Sels} + \textbf{Vit.} + Y_{S,O2}\, O_2 \rightarrow Y_{S,X}\, \textbf{Biom.} + Y_{S,CO2}\, CO_2 + Y_{S,H2O}\, H_2O + Y_{S,citric}\, \textbf{AC} + Y_{S,H}\, \Delta H$$

dans laquelle

Sub. désigne le glucose;
Sels désigne les sels ;
Vit. désigne les vitamines ;
$Y_{S,O2}$ désigne le rendement théorique limite de consommation de dioxygène par unité de substrat consommé ;
$O_2$ désigne le dioxygène ;
$Y_{S,X}$. désigne le rendement théorique limite de production de biomasse par unité de quantité de substrat consommé ;
$CO_2$ désigne le dioxyde de carbone ;
$Y_{S,CO2}$ désigne le rendement théorique limite de production de dioxyde de carbone par unité de quantité de substrat consommé ;
$Y_{S,H2O}$ désigne le rendement théorique limite de production d'eau par unité de quantité de substrat consommé ;
$H_2O$ désigne l'eau ;
$Y_{S,citric}$ désigne le rendement théorique limite de production d'acide citrique par unité de quantité de substrat consommé ;
AC désigne l'acide citrique
$Y_{S,H}$ désigne le rendement théorique limite de production d'enthalpie par unité de quantité de substrat consommé ;
$\Delta H$ désigne l'enthalpie de la réaction biologique ;

Avec un bilan carbone de la croissance avec synthèse de l'acide citrique répondant à l'équation 2 :

$$r_X^{net} + r_S^{net} + r_{citric}^{net} + r_{CO_2}^{net} = 0 \qquad \text{(Equation 2)}$$

dans laquelle

$r_X^{net}$ représente la vitesse nette de production de biomasse (levures) rapportée au volume total du système étudié (gramme de biomasse par unité de volume total et par unité de temps) ;

$r_S^{net}$ représente la vitesse nette spécifique de consommation de substrat rapportée au volume total du système étudié (gramme de substrat par unité de volume total et par unité de temps) ;

$r_{citric}^{net}$ représente la vitesse nette de production d'acide citrique rapportée au volume total du système étudié (gramme d acide par unité de volume total et par unité de temps) ;

$r_{CO_2}^{net}$ représente la vitesse nette de production de dioxyde de carbone rapportée au volume total du système étudié (gramme de dioxyde de carbone par unité de volume total et par unité de temps) ;

Pour plus de commodité pour les calculs des bilans élémentaires, toutes ces vitesses sont exprimées en Cmol.h$^{-1}$, La Cmole (abréviation : Cmol) est la mole ramenée à une mole de carbone. Par exemple, pour la mole de glucose de formule brute $C_6H_{12}O_6$ (6 carbone, 12 hydrogène, 6 oxygène) sa formule brute pour la Cmole est $C_1H_2O_1$ d'où

$$r_{CO_2}^{net} = -r_X^{net} - r_S^{net} - r_{citric}^{net} \qquad \text{(Équation 3)}$$

En associant à chaque composé i un degré de réduction $\gamma_i$, le bilan élémentaire généralisé s'écrit : (équation 4)

$$\sum_{j=1}^{m} r_j^{net} \cdot \gamma_j = 0 = r_{O_2}^{net} \cdot \gamma_{O_2} + r_X^{net} \cdot \gamma_X + r_S^{net} \cdot \gamma_S + r_{citric}^{net} \cdot \gamma_{citric} \qquad \text{(Equation 4)}$$

dans laquelle

$\sum_{j=1}^{m} r_j^{net} \cdot \gamma_j$ représente la somme des vitesses nettes des composés j multipliées par le degré de réduction du composé j ;

$r_{O_2}^{net}$ représente la vitesse nette de consommation de dioxygène rapportée au volume total du système étudié (gramme de dioxygène par unité de volume total et par unité de temps) ;

$\gamma_{O2}$ représente le degré de réduction du dioxygène ;

$r_X^{net}$ représente la vitesse nette de production de biomasse rapportée au volume total du système étudié (gramme d'acide par unité de volume total et par unité de temps) ;

$\gamma_X$ représente degré de réduction de la biomasse ;

$r_S^{net}$ représente la vitesse nette de consommation du substrat rapportée au volume total du système étudié (gramme de substrat par unité de volume total et par unité de temps) ;

$\gamma_S$ représente le degré de réduction du substrat ;

$r_{citric}^{net}$ représente la vitesse nette de production d'acide citrique rapportée au volume total du système étudié (gramme d'acide par unité de volume total et par unité de temps) et

$\gamma_{citric}$ représente le degré de réduction de l'acide citrique.

D'où on obtient l'équation 5

$$r_{O_2}^{net} = \frac{1}{\gamma_{O_2}} \cdot (- r_X^{net} \cdot \gamma_X - r_S^{net} \cdot \gamma_S - r_{citric}^{net} \cdot \gamma_{citric}) \qquad \text{(Equation 5)}$$

La combinaison des équations 3 et 5 permet d'exprimer la valeur du quotient respiratoire (QR) selon l'équation 6

$$QR = \frac{-r_X^{net} - r_S^{net} - r_{citric}^{net}}{\frac{1}{\gamma_{O_2}}(-r_X^{net}\cdot\gamma_X - r_S^{net}\cdot\gamma_S - r_{citric}^{net}\cdot\gamma_{citric})} \quad \text{(Equation 6)}$$

Selon la formule précédente, en quasi état d'équilibre, avec $r_X^{net}$ et $r_S^{net}$ constants, QR dépend de $r_{citric}^{net}$.

Si $r_{citric}^{net}$ devient positif (production d'acide citrique) alors QR diminue et si $r_{citric}^{net}$ devient négatif (consommation d'acide citrique comme substrat) alors, QR croit.

La valeur du QR est donc représentative du devenir du carbone consommé.

Des valeurs expérimentales de QR ont été déterminées au cours des différentes phases du métabolisme des levures en culture à savoir

- 1.01 pour la croissance à partir de glucose
- 1,11 pour l'accumulation de lipides à partir de glucose
- 0,90 pour la production d'acide citrique à partir de glucose
- 1,30 pour la croissance à partir d'acide citrique.

$\frac{r_C}{r_N}$, défini comme le rapport de la vitesse de consommation du carbone à la vitesse de consommation de l'azote, est un paramètre caractéristique du devenir du carbone intra cellulaire en l'absence de carbone résiduel accumulé dans le milieu de culture.

Au cours de la phase de croissance la formule de la biomasse est $C_1H_{1,86}O_{0,52}N_{0.16}$. alors que la formule de la composition moyenne en lipides accumulés est $C_1H_{1,93}O_{0,10}$.

Le temps de fonctionnement de la pompe d'alimentation en composé basique est un paramètre clé qui révèle la production d'acide citrique.

Une base (KOH par exemple), ajoutée au milieu de culture, permet de neutraliser l'acidité apporté par les sels d'alimentation (pH <1) et, éventuellement, les acides produits par les microorganismes.

Quand un excès de carbone par rapport aux flux d'azote intervient, il y a production d'acide citrique, et le dispositif de contrôle et régulation de pH met en route la pompe d'alimentation en composé basique.

Comme le débit d'alimentation en sels est proportionnel à la vitesse de consommation du glucose, et que la consommation du glucose est une fonction de la vitesse de croissance de la levure, alors le temps de fonctionnement de la pompe d'alimentation en composé basique varie en fonction de la vitesse de croissance microbienne

Comme les levures nécessitent moins de sels et d'oligo-éléments au cours de la phase d'accumulation (renouvellement cellulaire minimum, faible activité enzymatique), le rapport sels / débit de carbone peut être augmenté de 10 (valeur en phase de croissance) à 60 (valeur en phase d'accumulation de lipides).

Le temps de fonctionnement de la pompe de base pour la neutralisation des sels d'alimentation est négligeable devant le temps de fonctionnement de la pompe d'alimentation en composé basique pour la neutralisation de l'acide citrique produit par la levure. Le temps de fonctionnement de la pompe de base peut donc être considéré comme un paramètre pertinent pour la détection de la production d'acide citrique. Le temps de fonctionnement de la pompe d'alimentation en composé basique est un paramètre mesuré en ligne par la détection du contact marche/arrêt de la pompe.

Les mesures du temps de fonctionnement de la pompe d'alimentation en composé basique permet de calculer une augmentation dudit temps détectant ainsi une production d'acide citrique.

Le tableau suivant représente différentes situations du taux spécifique de croissance et l'action sur la pompe d'alimentation en substrat (PalS) combinant QR, $\frac{r_C}{r_N}$ et la variation du temps de fonctionnement de la pompe d'alimentation en composé basique ($\Delta$tPB).

| | Taux de croissance | Taux de lipides | Taux d'acide citrique | $\Delta$QR | $\Delta \frac{r_C}{r_N}$ | $\Delta$tPB | action sur Pals |
|---|---|---|---|---|---|---|---|
| 1 | $\mu \geq \mu_N$ | $q_{lipides} > 0$ | $q_{citrique} > 0$ | ↓ | ↑ | ↑ | ↓ |
| 2 | $\mu = \mu_N$ | $q_{lipides} > 0$ | $q_{citrique} = 0$ | ↑ | ↑ | → | → |
| 3 | $\mu \leq \mu_N$ | $q_{lipides} = 0$ | $q_{citrique} = 0$ | → | ↓ | → | ↑ |
| 4 | $\mu \leq \mu_N$ | $q_{lipides} = 0$ | $q_{citrique} \leq 0$ | ↑ | ↓ | → ou ↓ | ↑ |
| 5 | $\mu \leq \mu_N$ | $q_{lipides} < 0$ | $q_{citrique} = 0$ | ↓ | ↓ | → ou ↓ | ↑ |
| $\mu$ [$h^{-1}$] représente le taux de croissance spécifique contrôle par le flux de carbone, $\mu_N$ représente le taux de croissance spécifique contrôlé par le flux d'azote [$h^{-1}$]. | | | | | | | |

- En 1, $\mu \geq \mu_N$, $q_{lipides} > 0$ : la croissance et la synthèse des lipides ne sont pas limitées par le flux de carbone,

  $q_{citric} > 0$ du fait de l'excès de carbone
  La production d'acide citrique induit une diminution de QR ;
  L'accumulation de lipides et la production d'acide citrique induisent une augmentation de $\frac{r_C}{r_N}$ ;

  Comme le pH est régulé, l'acide citrique est neutralisé grâce à une augmentation du temps de fonctionnement de la pompe d'alimentation en composé basique.
  Le débit de la pompe d'alimentation en substrat est trop élevé, le rôle du contrôleur et régulateur est de diminuer son débit.
- En 2, $\mu = \mu_N$, $q_{lipides} > 0$ : la croissance et la synthèse des lipides ne sont pas limitées par le flux de carbone.

  $q_{citric} = 0$ il n'y a pas eu d'excès de carbone.

La synthèse des lipides intervenant, QR peut atteindre la valeur de 1,11 et $\frac{r_C}{r_N}$ augmente.

Comme $q_{citric} = 0$, le temps de fonctionnement de la pompe d'alimentation en composé basique est constant.

Le débit de la pompe d'alimentation en substrat est optimal, le contrôleur n'a pas à intervenir.

- En 3, $\mu \leq \mu_N$ la croissance est limitée par le flux de carbone (il n'y avait ni synthèse de lipides, ni synthèse d'acide citrique) et une augmentation de flux de carbone entraîne une augmentation du taux de croissance ($\mu > \mu_N$), mais le flux de carbone n'est pas suffisant pour permettre la synthèse des lipides ;

  $q_{citric} = 0$, QR ne varie pas, mais $\frac{r_C}{r_N}$ diminue parce que les flux de carbone sont plus élevés lors de la synthèse catalytique que de la synthèse des lipides en comparaison avec les flux optimaux.
  Comme $q_{citric} = 0$, le débit de la pompe d'alimentation en substrat est constant.
  Le débit de la pompe d'alimentation en substrat est trop faible, le contrôleur et régulateur doit alors augmenter le débit

- En 4, la croissance est limitée par le flux de carbone ; $\mu \leq \mu_N$ de ce fait, le flux de carbone n'est pas suffisant pour permettre une accumulation de lipides ($q_{lipids} = 0$) et pour la synthèse de l'acide citrique ($q_{citric} = 0$).
  L'acide citrique pourrait même être consommé par la levure ($q_{citric} \leq 0$) induisant une augmentation du QR, une diminution de $\frac{r_C}{r_N}$ et l'activation de la pompe d'alimentation en composé acide.
  Le débit de la pompe d'alimentation en substrat est trop faible, le contrôle doit augmenter le débit de ladite pompe.
- En 5, la culture est en phase de croissance.

La croissance est limitée par le flux de carbone ;

$\mu < \mu_N$ et les lipides stockés peuvent être consommés par les levures ($q_{lipids} \leq 0$), avec une diminution de $\frac{r_C}{r_N}$ et de QR.

Le débit de la pompe d'alimentation en substrat est trop faible, le contrôleur et régulateur doit augmenter le débit de ladite pompe.

Ces différentes phases peuvent être visualisées selon la Figure 1 qui représente les différentes phases du métabolisme de *Yarrowia lipolytica* en fonction du débit de la pompe d'alimentation en substrat.

Au cours de la phase d'accumulation de lipides, la part azote diminue quand la part de lipides cellulaires augmente. Mais l'accumulation de l'acide citrique peut conduire à un rapport C / N trop élevé.

Ainsi, afin de distinguer les trois cas : "croissance", "croissance avec accumulation de lipides" et "croissance avec accumulation de lipides et synthèse d'acide citrique", les trois paramètres QR, $\frac{r_C}{r_N}$ et le temps de fonctionnement de la pompe d'alimentation en composé basique sont nécessaires.

Il est proposé d'utiliser une combinaison entre QR et $\frac{r_C}{r_N}$ de la façon suivante :

$$\frac{\frac{r_C}{r_N}}{QR} = k * \frac{r_{O_2}}{r_N} \quad \text{(Equation 7)}$$

Où k correspond à $\frac{r_C}{r_{CO_2}}$ équivalent à $Y_{S,CO2,}$ rendement théorique limite de production de dioxyde de carbone par unité de quantité de substrat consommé, constant pour un métabolisme stabilisé (accumulation de lipides, croissance).

Par conséquent, au court de la phase d'accumulation des lipides, afin d'optimiser cette accumulation tout en limitant au maximum (voir annihilant) la production d'acide citrique dans la culture, $\frac{r_{O_2}}{r_N}$ et le temps de fonctionnement de la pompe d'alimentation en composé basique peuvent être intégrés comme paramètres de l'algorithme de contrôle et régulation avec pour objectif une optimisation de $\frac{r_C}{r_N}$ et de QR.

Ainsi les inventeurs ont pu établir l'algorithme de commande du contrôleur et régulateur dérivé d'un PID.

Les travaux des inventeurs ont conduit à l'élaboration d'un algorithme de commande original : le calcul des actions proportionnelle, intégrale et dérivée a été effectué d'après les équations décrites ci-après en utilisant principalement les variables disponibles en ligne suivantes :

- masse de la solution d'alimentation en source de carbone, avantageusement du glucose seul, du glucose et du glycérol ou du glycérol seul,
- composition et débits du gaz d'entrée et du gaz de sortie
- temps de fonctionnement de la pompe de base à un débit constant (mesuré hors circuit).

Différents gains, tolérances et valeurs critiques ont été calculés et les facteurs Proportionnel, Intégral et Dérivé ont été estimés d'après les corrélations suivantes : la première étape correspond au calcul du "Point de consigne" $O_2$/N avant ajustage

$$\left(\frac{O_2}{N}\right)_1 = \frac{\sum_{i=0}^{n}\left(\frac{O_2}{N}\right)_i}{n} \quad \text{(Equation 9)}$$

Équation dans laquelle $\left(\frac{O_2}{N}\right)_i$ représente le rapport $\frac{r_{O2}}{r_N}$ mesuré au temps i ; et

n représente le nombre de mesures fixé arbitrairement

et l'écart-type de n mesures de $\frac{r_{O2}}{r_N}$ sans ajustage était

$$SD_1 = \sqrt{\frac{n\sum_{i=0}^{n}\left(\frac{O_2}{N}\right)_i - \left(\sum_{i=0}^{n}\left(\frac{O_2}{N}\right)_i\right)^2}{n(n-1)}} \qquad \text{(Equation 10)}$$

Les mesures hors de l'intervalle [$X_1$ - $SD_1$ * Cr ; $X_1$ + $SD_1$ * Cr] ont été exclues. Le nombre de mesure restante est nommé n'. La moyenne $X_2$ et l'écart-type $SD_2$ ont été calculés avec les n' mesures retenues, Cr est un coefficient critique défini par l'utilisateur, il permet d'élargir ou de rétrécir l'intervalle.

Si $X_2$ est compris dans l'intervalle [$X_2$ - $SD_2$ * $K_{DB}$; $X_2$ + $SD_2$ * $K_{DB}$] (où $K_{DB}$ est le coefficient Dead Band défini par l'utilisateur, il permet d'élargir ou de rétrécir l'intervalle), c'est-à-dire dans l'intervalle de l'écart à la consigne acceptable, alors $\Delta m = 0$ ; dans les autres cas, l'écart par rapport au point de consigne (l'erreur) est calculé comme suit :

$$\Delta m = \left(\frac{O_2}{N}\right)_{Sp} - \left(\frac{O_2}{N}\right)_2 \qquad \text{(Equation 11)}$$

équation dans laquelle

$\Delta m$ représente l'erreur

$\left(\frac{O_2}{N}\right)_{Sp}$ représente la consigne de $\frac{r_{O2}}{r_N}$

Le système biologique étant pas essence un système auto catalytique, l'intensité des signaux augmente avec l'avancement de la réaction Afin de prendre en considération la dynamique des phénomènes biologiques il est préférable de multiplier cette intensité à l'unité catalytique, la biomasse, dont la concentration n'est pas disponible en ligne. Il apparaît que pour les systèmes biologiques, le rendement enthalpique est à peu près constant quelque soit la culture et le microorganisme mis en jeu, et ce, pour les substrats de degré de réduction inférieur à 4,5. Ceci correspond à un rendement carbone prévisible, et sauf cas limite d'une oxydation quasi-totale du substrat par réaction de maintenance, à des rendements variant très peu en cours de culture pour un type de microorganisme donné. Ainsi on peut avoir une bonne estimation de l'avancement de la réaction par la quantité de dioxyde de carbone produite, et à un rendement près, la quantité de biomasse accumulée au cours de la fermentation. Ainsi en multipliant les signaux par la quantité de $CO_2$, la réponse correctrice sera d'autant plus adapté à la taille (augmentation de la biomasse proportionnelle à la quantité de $CO_2$ produit) dynamique (les vitesses de réactions sont proportionnelles à la quantité de biomasse présente) du système asservi.

L'action proportionnelle au temps t est ensuite définie par :

$$P(t) = \Delta m * K_P * r_{CO2} \qquad \text{(Equation 12)}$$

Equation dans laquelle

- $K_P$ représente le facteur proportionnel (c'est un facteur correctif ajustable par l'utilisateur) ;
- $r_{CO2}$ représente la vitesse de production dioxyde de carbone rapportée au volume total.

L'action intégrale au temps t est calculée au moyen de l'équation 13 suivante :

$$I(t) = (I(t - 1) + \Delta m) = K_i * r_{CO_2} \quad \text{(Equation 13)}$$

Equation dans laquelle

- (I(t - 1) représente l'action intégrale au temps t moins une unité de temps ;
- $K_i$ représente le facteur intégral (c'est un facteur correctif ajustable par l'utilisateur).

L'action dérivé se démarque des actions dérivés des procédés chimiques : en effet, elle ne prend pas en compte la dérivé de l'écart à la consigne. L'action dérivée est liée à la pente a(t) de la courbe (temps de fonctionnement de la pompe d'alimentation en composé basique) = fonction de (t) dans une fenêtre de temps glissante.

$$D(t) = a(t) * r_{CO_2} * K_d \quad \text{(Equation 14)}$$

Equation dans laquelle
$K_d$ représente le facteur dérivé (c'est un facteur correctif ajustable par l'utilisateur) ; Si a(t) = 0 ; alors D = 0.
Et l'action PID(t) qui représente la valeur de sortie de l'algorithme de commande avec P(t) qui représente le facteur proportionnel, I(t) qui représente le facteur intégral et D(t) qui représente le facteur dérivé au temps t, s'établit

$$PID(t) = \Delta m * Kp * rCO2 + (I(t - 1) + \Delta m) * Ki * rCO2 + a(t) * rCO2 * Kd \quad \text{(Equation 15)}$$

L'unité de PID(t) est une combinaison de molCO2.s-1 molO2.molN-1 et s.s-2, désignée par [u].
Pour simplifier la description de l'algorithme, différents autres paramètres sont utilisés, tels que le coefficient de rejet, le facteur de réduction de PID, la tolérance supérieure, la tolérance inférieure, le gain proportionnel, la coefficient de zone inerte, le gain dérivé, le gain dérivé critique, le gain intégral, la valeur de sortie de commande critique et dérivée critique.
Le réglage de la boucle de contrôle et la recherche de points de consigne optimaux ont été déterminés au cours d'expériences préliminaires en mode fed-batch.
Ainsi l'invention a aussi pour objet un procédé de culture de Yarrowia lipolytica, caractérisé en ce que le rapport entre le taux de consommation de carbone et le taux de consommation de l'azote (rC/rN) ou le taux de consommation de carbone et le taux de consommation de l'azote (rC/rN) et le taux de consommation de l'oxygène et le taux de consommation de l'azote (ro2/rN), avantageusement le taux de consommation de carbone et le taux de consommation de l'azote (rC/rN) et le taux de consommation de l'oxygène et le taux de consommation de l'azote (ro2/rN), dans la culture est maintenu constant au travers d'un contrôleur d'un type PID dont le signal de sortie est régi par l'équation suivante

$$PID(t) = \Delta m * Kp * rCO2 + (I(t - 1) + \Delta m) * Ki * rCO2 + a(t) * rCO2 * Kd \quad \text{(Equation 16)}$$

L'invention, a également pour objet un dispositif de culture de Yarrowia lipolytica comprenant au moins un fermenteur muni des moyens d'alimentation en éléments nécessaires à la culture de levures du genre Yarrowia, particulièrement de la souche Yarrowia lipolytica et d'un moyen de contrôle de la valeur du rapport entre la vitesse de consommation de carbone et la vitesse de consommation de l'azote (rC/rN), ou du rapport entre la vitesse de consommation de carbone et la vitesse de consommation de l'azote (rC/rN) et le coefficient respiratoire (QR) ou du rapport entre la vitesse de consommation de carbone et la vitesse de consommation de l'azote (rC/rN) et le rapport entre la vitesse de consommation d'oxygène et la vitesse de consommation d'azote (rO2/rN) ou du rapport entre la vitesse de consommation d'oxygène et la vitesse de consommation d'azote (rO2/rN) et QR, , caractérisé en ce que le moyen de maintenir aux valeurs fixées du rapport entre la vitesse de consommation de carbone et la vitesse de consommation de l'azote (rC/rN), ou du rapport entre la vitesse de consommation de carbone et la vitesse de consommation de l'azote (rC/rN) et le coefficient respiratoire (QR) ou du rapport entre la vitesse de consommation de carbone et la vitesse de

consommation de l'azote (rC/rN) et le rapport entre la vitesse de consommation d'oxygène et la vitesse de consommation d'azote (rO2/rN) ou du rapport entre la vitesse de consommation d'oxygène et la vitesse de consommation d'azote (rO2/rN) et QR est un contrôleur de type PID tel que décrit précédemment.

D'autres caractéristiques de l'invention pourront apparaître à la lecture des exemples qui suivent qui illustrent la présente demande sans toutefois la limiter ainsi que des figures annexées dans lesquelles :

- la Figure 1 représente les différentes phases du métabolisme de *Yarrowia lipolytica* en fonction du débit de la pompe d'alimentation en substrat ;
- les Figures 2 et 3 représentent les 3 phases d'une culture de *Yarrowia lipolytica* dans les deux modes de cultures possibles :

  - Figure 2 : alimentation discontinue sans contrôleur type PID (alimentation discontinue 1) et
  - Figure 3 : alimentation discontinue avec contrôleur type PID (alimentation discontinue 2),

dans lesquelles, en fonction du temps, exprimé en heures

-♦- représente la concentration en substrat exprimés en $Cmol_S.L^{-1}$;
-■- représente la concentration en biomasse exprimée en $Cmol_x.L^{-1}$ ;
-x- représente la concentration en azote exprimée en $Nmol.L^{-1}$.

- la Figure 4 représente la variation de la concentration en acide citrique [$Cmol_{citric}.L^{-1}$] au cours du temps dans des cultures avec et sans contrôleur de type PID dans laquelle, en fonction du temps exprimé en heures :

-●- représente la concentration en acide citrique exprimée en $Cmol.L^{-1}$ sans contôle PID;
-+- représente la concentration en acide citrique exprimée en $Cmol.L^{-1}$ avec contôle PID.

- la Figure 5 représente le taux d'accumulation en lipides intracellulaires [$Cmol_{lip}.Cmol_x$] au cours du temps dans des cultures avec et sans contrôleur type PID dans laquelle, en fonction du temps exprimé en heures :

-○- représente le pourcentage de lipides exprimés en $Cmol_{lipides}.Cmol_x^{-1}$avec contrôle PID ;
-●- représente le pourcentage de lipides exprimés en $Cmol_{lipides}.Cmol_x^{-1}$sans contrôle PID.

- la figure 6 représente le profil des lipides accumulés au cours de cultures en mode alimentation discontinue (Fed-Batch) de *Yarrowia lipolytica* sur milieu synthétique et glucose, avec commande de type PID durant la phase d'accumulation des lipides dans laquelle:

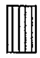 représente la teneur en différents acides gras par rapport à la totalité des acides gras présents dans les lipides cellulaires, exprimée en $Cmol_{AGi}.Cmol_{AGtotaux}^{-1}$ sans contrôle PID ;

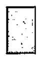 représente la teneur en différents acides gras par rapport à la totalité des acides gras présents dans les lipides cellulaires exprimée en $Cmol_{AGi}.Cmol_{AGtotaux}^{-1}$ avec contrôle PID.

- la Figure 7 représente la vitesse spécifique de production de lipides spécifiques, la vitesse spécifique de production d'acide citrique et le signal PID(t) exprimés respectivement en $Cmol_{lipides}.Cmol_X^{-1}.h^{-1}$, en $Cmol_{citrique}.Cmol_X^{-1}.h^{-1}$ et en u au cours de culture de *Yarrowia lipolytica* avec commande de type PID ;

- la Figure 8 représente les variations du coefficient respiratoire (QR, sans dimension) et du rapport $\frac{r_C}{r_N}$ en fonction du temps exprimé en heures, au cours de culture de *Yarrowia lipolytica* sans commande de type PID dans laquelle :

-●- représente le rapport rC/rN et
--- représente le coefficient respiratoire.

- la Figure 9 représente les variations du coefficient respiratoire (QR, sans dimension) et du rapport $\frac{r_C}{r_N}$ ($Cmol.Nmol^{-1}$) en fonction du temps exprimé en heures, au cours de culture de *Yarrowia lipolytica* avec commande de type PID dans laquelle :

-●- représente le rapport rC/rN et
--- représente le coefficient respiratoire.

- la Figure 10 représente les variations des flux de la pompe d'alimentation en substrat [$Cmol_S.Cmol_X^{-1}.L^{-1}$] et du volume de composé basique ajouté en fonction du temps exprimé en heures au cours de culture avec ou sans commande de type PID dans laquelle

-●- représente le rapport rC/rN et
--- représente le coefficient respiratoire
-○- représente le volume de base ajouté sans PID
-♦- représente le volume de base ajouté avec PID -

- la Figure 11 représente les variations du signal PID (u) et du débit de glucose ($mL.h^{-1}$) en fonction du temps exprimé en heures dans laquelle

-x- représente le débit de glucose et
-●- représente les variations du signal PID.

- la Figure 12 représente l'évolution du quotient respiratoire (QR, [$Cmol_{CO2,}Cmol_{O2}^{-1}$]) lors d'une culture Y *lipolytica* à 28 °C, pH 5,5 en co-substrat glucose/glycérol (les flèches représentent les événements de début de limitation azote, de début d'alimentation en glycérol et de début de mise en marche du contrôleur).
La Figure 13 représente l'évolution de la concentration en biomasse totale ([X], [$Cmol_X.L^{-1}$]) et de la teneur en lipides totaux (%lip, [$Cmol_{lip}.Cmol_X^{-1}$]) lors d'une culture Y *lipolytica* à 28°C, pH 5,5 en double substrat glucose/glycérol avec utilisation du contrôleur sur substrat glycérol pur.

-□- représente la quantité de biomasse
-○- représente la teneur en lipide.

La Figure 14 représente l'évolution des concentrations en glucose, glycérol et acide citrique dans le surnagent ([glucose], [glycérol], [acide citrique], [$Cmol,L^{-1}$]) lors d'une culture *Y. lipolytica* à 28 °C, pH 5,5 en co-substrat glucose/glycérol.

-▲- représente la concentration en acide citrique exprimée en Cmol./L
-□- représente la concentration en glucose exprimée en Cmol./L
-|- représente la concentration en glycérol exprimée en Cmol./L.

Exemples

A) Matériels et méthodes

La souche sauvage de *Yarrowia lipolytica* a été fournie par JM NICAUD, *Laboratoire de Microbiologie et Génétique Moléculaire, CNRS, UMR* 2585, *INRA, UMR1238, AgroParisTech, INRA centre de Grignon F-78850 Thiverval-Grignon, France.*

Des stocks de souche ont été réalisés à partir de précultures axéniques réalisées dans des Erlenmeyers bafflés placés sur une table agitante, avec un milieu riche ayant une concentration initiale en glucose de 10 $g.l^{-1}$. En milieu de phase exponentielle, des échantillons de 1 mL ont été prélevés et additionnés de glycérol stérile (30 % en volume/volume). Puis ces stocks ont été conservés dans des flacons stériles à -80°C. Ces cultures concentrées congelées ont été utilisées pour ensemencer les différentes précultures à des fins de culture en mode alimentation discontinue.

Les précultures de levure ont été effectuées dans deux fioles d'Erlenmeyer de 100 ml contenant 8 ml de milieu riche LB à 30°C pendant 16 h sur une table d'agitation rotatif (100 tr/min). Les cultures ont été transférées dans deux fioles d'Erlenmeyer de 250 ml contenant 72 ml de milieu minéral (pH 5,6) ayant une concentration initiale en glucose de 10 $g.l^{-1}$. Après 12 h à 30°C, les cultures d'un volume de 80 ml ont été utilisées pour ensemencer deux fioles d'Erlenmeyer de 5 l contenant 710 ml de milieu minéral avec des vitamines et ont été placées à incuber à 30°C pendant 12 h, avec une concentration initiale en glucose de 10 $g.l^{-1}$. Le contenu d'une des fioles de la dernière culture a été utilisé pour ensemencer 8 l du même milieu minéral dans un bioréacteur de 20 l. La série de précultures réalisées en parallèle est utilisé pour vérifier la reproductibilité des précultures.

La composition du milieu minéral était la suivante : $K_2HPO_4$ : 3 $g.l^{-1}$ ; $(NH_4)_2SO_4$ : 3 $g.L^{-1}$ ; $NaH_2PO_4,H_2O$ : 3 $g.l^{-1}$ ; $MgSO_4,7H_2O$ : 1 $g.l^{-1}$ ; $ZnSO_4,7H_2O$ : 0,04 $g.l^{-1}$ ; Fe $SO_4,7H_2O$ : 0,0163 $g.l^{-1}$ ; $MnSO_4,H_2O$ : 0,0038 $g.l^{-1}$ ;

$CoCl_2,6H_2O$ : 0,0005 $g.l^{-1}$ ; $CuSO_4,5H_2O$ : 0,0009 $g.l^{-1}$ ; $Na_2MoSO_4,2H_2O$ : 0,00006 $g.l^{-1}$ ; $CaCl_2,2H_2O$ : 0,23 $g.l^{-1}$ ; $H_3BO_3$ : 0,03 $g.l^{-1}$ ; et 10 ml de solution de vitamines. La solution de vitamines a été préparée à une concentration d'un facteur 1000 : d-biotine : 0,05 $g.l^{-1}$, chlorhydrate de thiamine : 1 $g.l^{-1}$, acide panthoténique : 1 $g.l^{-1}$ ; chlorhydrate de pyridoxol : 1 $g.l^{-1}$ ; acide nicotinique : 1 $g.l^{-1}$, acide p-aminobenzoïque : 0,2 $g.l^{-1}$, myo-inositol : 25 $g.l^{-1}$. Avant stérilisation, le pH de ce milieu a été ajusté à 4,5 avec une solution de $H_3PO_4$ et à pH de travail (5,5) avec une solution d'ammoniac.

• *Cultures*

Des cultures en mode alimentation discontinue (8 l) ont été effectuées dans un bioréacteur de 20 l en utilisant le système de culture Braun Biostat E (Braun, Melsungen, Allemagne) sans limitation d'oxygène.

La température a été régulée à 28°C et le pH a été régulé à 5,5 par addition d'une solution à 10 $mol.l^{-1}$ de $NH_3$ (phase de croissance) ou d'une solution de KOH (phase d'accumulation de lipides). Le fermenteur a été relié à un ordinateur. Un logiciel, élaboré dans le laboratoire des inventeurs, a permis l'acquisition directe des paramètres tels que vitesse d'agitation, pH, température, pression partielle d'oxygène dissous (DO), addition des bases et de l'agent anti-mousse, pour le contrôle et la régulation de ces paramètres.

La pression dans le bioréacteur a été régulée à 0,3 bar (pression relative). La quantité maximale d'agent antimousse (Struktol) ajoutée est égale à 0,5 ml. Le bioréacteur est équipé de trois systèmes d'alimentation stériles (source de carbone, avantageusement glucose seul, glucose et glycérol ou glycérol seul, sel, ammoniaque ou hydroxyde de potassium) utilisant des pompes péristaltiques (Masterflex et Gilson). La concentration d'alimentation en source de carbone, avantageusement du glucose seul, du glucose et du glycérol ou du glycérol seul est égale à 730 $g.l^{-1}$.

La masse de la solution de source de carbone, avantageusement du glucose seul, du glucose et du glycérol ou du glycérol seul et de la solution d'ammoniac (ou d'hydroxyde de potassium) introduite dans le bioréacteur est mesurée de manière continue par le suivi des masses des flacons stock des solutions (balance de marque Sartorius). Les concentrations en source de carbone, avantageusement du glucose seul, du glucose et du glycérol ou du glycérol seul et en azote dans le fermenteur sont estimées d'après l'équation des bilans de carbone et redox. Le débit d'évaporation est estimé à partir de la température de culture, de l'efficacité du condenseur du fermenteur et du débit d'aération. Le volume de culture est calculé d'après un bilan de matière réalisé à partir des apports en substrat, sel, ammoniaque, base, vitamines et agent antimousse et des sorties par évaporation, échantillonnage avec et sans biomasse.

• *Agents chimiques*

Les produits chimiques (sels, oligoéléments, acide orthophosphorique et $NH_3$) ont été fournis par Prolabo (France), et les vitamines ont été fournies par Sigma (E.U.A.). Tous ces produits sont de la plus haute qualité analytique disponible. Le glucose est fourni par Roquette (France).

• *Stratégie d'alimentation en glucose*

Au cours de la phase de croissance, un profil exponentiel du débit de la pompe d'alimentation en source de carbone, avantageusement en glucose seul, en glucose et en glycérol ou en glycérol seul permet le maintien d'un taux de croissance constant.

Au cours de la phase d'accumulation, un algorithme de commande du débit de pompe d'alimentation en substrat est utilisé afin d'apporter un flux de carbone répondant aux besoins de croissance résiduelle (en fonction du débit d'alimentation en ammonium) et la néo-synthèse des lipides sans production d'acide citrique. L'explication de l'opération de commande se trouve dans la partie "algorithme de commande".

• *Stratégie d'alimentation en sels concentrés*

Le bioréacteur est alimenté par un débit de solution de sels concentrés correspondant à 1/10 du débit d'alimentation en substrat. La composition de la solution de sels concentrés est la suivante : KCl : 20 $g.l^{-1}$, $CuSO_4,5H_2O$ : 0,6 $g.l^{-1}$, NACI : 20 $g.l^{-1}$, $Na_2MoO_4,2H_2O$ : 0,094 $g.l^{-1}$, $MgSO_4,7H_2O$ : 27 $g.l^{-1}$, $CaCl_2,2H_2O$: 6,4 $g.l^{-1}$, $ZnSO_4,7H_2O$ : 7,7 $g.l^{-1}$, $FeSO_4,7H_2O$ : 3,97 $g.l^{-1}$, $MnSO_4,H_2O$ : 0,47 $g.l^{-1}$, $H_3BO_3$ : 0,3 $g.l^{-1}$, $CoCl_2,6H_2O$ : 0,3 $g.l^{-1}$, $H_3PO_4$ : 46,7 $g.l^{-1}$.

• *Stratégie d'alimentation en vitamines*

Toutes les cultures sont effectuées par une alimentation séquencée en vitamines en fonction du taux de croissance : des quantités de 0,1 % (vol/vol) de solution de vitamines sont ajoutées lors de production de 10 $g.l^{-1}$ de biomasse.

• *Stratégie d'alimentation en ammonium*

Au cours de la phase de croissance, l'azote est ajouté à l'aide de la pompe de base pour la régulation de pH à la valeur constante égale à 5.5. Au cours de la phase de production de lipides, l'azote est ajouté par une pompe péristaltique avec un débit linéaire de solution de $NH_3$ (5 $mol.l^{-1}$) afin de maintenir une vitesse de croissance spécifique constante et le pH est régulé par apport d'une solution de KOH (10 $mol.l^{-1}$).

• *Méthodes analytiques*

La concentration en levure est évaluée par des mesures spectrophotométriques à 600 nm dans un spectrophotomètre HITACHI U-1100 dans une cellule en quartz ayant un trajet optique de 0,2 cm. Des dilutions de l'échantillon sont effectuées de telle sorte que la densité optique soit comprise dans la plage de 0,1 à 0,6 UA. Pour chaque échantillon, la moyenne de trois mesures est calculée. Pour la détermination de la masse sèche des cellules, des échantillons de culture (5 à 10 ml) sont recueillis par filtration sur une membrane de 0,45 $\mu$m (Sartorius) et sont séchés à 200 mm d'Hg et 60°C pendant 48 h jusqu'à obtention d'une masse constante. La quantité de cendre est déterminée après deux combustions totales des filtres de masse sèche avec biomasse en présence de 200 $\mu$l de solution à 20 $g.l^{-1}$ de $NH_4NO_3$ dans un four à moufle à 550°C pendant 12 h à chaque fois. La formule de la biomasse a été déterminée à l'ENSIACET (Toulouse, France) par analyse élémentaire de C, H, O et N et des cendres. En raison d'une accumulation importante de lipides, la formule de la biomasse varie au cours de la culture de $CH_{1,86}O_{0,52}N_{0,16}$ (phase de croissance) à $CH_{2,00}O_{0,59}N_{0,07}$ (phase d'accumulation).

Toutes les 20 min, un échantillon de surnageant est recueilli par un système de filtration tangentielle associé à un collecteur automatique de fractions. Un échantillon de milieu de culture est recueilli chaque heure directement à travers un septum. Tous les échantillons sont stockés à -20°C.

La détermination des concentrations en alcools, en acides organiques et en sucres des surnageants est effectuée par HPCL en utilisant une colonne Aminex HPX-87H+ (300 mm x 7,8 mm), dans les conditions suivantes : température de 50°C, $H_2SO_4$ 5 $mmol.L^{-1}$ comme éluant (débit de 0,5 $ml.min^{-1}$) et détection double (réfractomètre et UV à 210 nm). Les composés sont identifiés et quantifiés par référence à des solutions pures de chaque composé. La détermination de la concentration en glucose à partir des surnageants de culture est également effectuée au cours de la culture au moyen d'un analyseur de glucose YSI modèle 27 A ; Yellow Springs Instruments, Yellow Springs, Ohio.

Une électrode à ion ammonium (PH/ISE meter modèle 710A + électrode de captage de gaz ammoniac modèle 95-12, Orion Research Inc Boston, E.U.A.) est utilisée pour quantifier la concentration résiduelle en ammoniaque dans le milieu de culture.

L'analyse du gaz à la sortie est effectuée, toutes les 20 secondes, par spectroscopie de masse en sortie du condenseur de gaz du fermenteur. Le spectromètre de masse (PRIMA 600s ; VG Gas, Manchester, Royaume-Uni) est utilisé en raison de sa précision pour mesurer les compositions en $CO_2$, $O_2$, $N_2$ et Ar.

La vitesse de consommation d'$O_2$ et la vitesse de production de $CO_2$ sont calculées d'après les bilans de matière, combinant le volume du gaz dans le réacteur, le débit d'air entrant (mesuré par un débitmètre massique), la température, l'humidité et la pression et la composition des gaz d'entrée et de sortie

Les cellules sont observées avec un microscope optique (Olympus BH2, objectif : DplanApoUVPL et DA po 100 UV PL) équipé d'une caméra CCD (DXM 1200, Nikon).

Les images sont numérisées en un réseau de 977_715 pixels par un système de capture d'image (Matrox 975-0201). Pour chaque échantillon, 20 images et un nombre total d'au moins 200 cellules sont traités en utilisant le logiciel commercial d'analyse d'images LUCIA 4.6 de Laboratory Imaging Ltd. (République Tchèque).

L'extraction des lipides cellulaires totaux est effectuée selon la technique de Cot M. et al. (2005) qui est une modification du mode opératoire de Bligh et Dyer, comme suit : L'extraction au gradient de solvant se réalise dans des tubes pyrex en verre avec des bouchons à vis siliconés recouverts de téflon placés sur un agitateur à rouleaux sous agitation de 50 RPM. 500 mg de lyophilisats sont placés dans les tubes avec le mélange de solvants. Le solvant enrichi en extrait lipidique est récupéré par centrifugation (5000 g ; 4°C ; 10 min).

Les cellules sont remises en présence d'un nouveau mélange de solvants. 3 mélanges successifs sont utilisés : méthanol/chloroforme (2:1, vol/vol), (1:1, vol/vol) et enfin (1:2, vol/vol). Chaque étape d'extraction consiste en une incubation pendant 24 h à température ambiante sur un mélangeur par roulement.

Les quatre phases organiques sont mélangées et lavées deux fois avec une solution à 25 % (vol/vol) de solution à 0,88 % (masse/volume) refroidie par de la glace. Comme l'extraction pourrait "extraire" les contaminants non lipidiques (sucres, aminoacides, protéines et sels), la phase organique a été confrontée à une solution de KCl (0,08 $g.l^{-1}$) pendant 15 min sous agitation douce et une technique de séparation liquide/liquide a été ensuite utilisée après centrifugation (5000 x g, 10 min).

Finalement, les lipides ont été recueillis après l'évaporation des solvants dans un évaporateur rotatif (35°C, 50 mbar). La teneur totale en lipides a été quantifiée par une méthode gravimétrique. L'extrait de lipides a été maintenu

dans un mélange chloroforme/méthanol à -20°C.

Les acides gras libres ou liés ont été méthylés en ester méthylique d'acides gras (FAME) en utilisant de l'hydroxyde de triméthylsulfonium (TMSH, 0,2 M dans le méthanol, Macherey-Nagel, Allemagne). L'analyse GC a été effectuée sur un appareil de chromatographie en phase gazeuse Hewlett-Packard 5890 équipé d'un colonne en silice fondue WCOT de 50 m x 250 μm x 25 μm (VARIAN, E.U.A.) et d'un FID, dans les conditions suivantes : phase mobile : $N_2$, débit 50 $ml.min^{-1}$, température du four : 50-75°C à 9°$C.min^{-1}$, puis 75-140°C à 13°$C.min^{-1}$, puis 140-180°$C.min^{-1}$ à 1,5°$C.min^{-1}$, puis 180-240°C à 4,5°$C.min^{-1}$, température d'injecteur 140°C, température du détecteur 250°C.

L'identification des esters méthyliques est basée sur la comparaison des temps de rétention avec ceux de substances d'étalonnage connues. Les étalons internes consistent en C9:0 pour la courte longueur de chaîne carbonée des acides gras et C19:0 pour la longueur moyenne de chaîne carbonée.

Le tableau suivant résume les valeurs des paramètres pour le mode du système de commande type PID après 60h de culture au cours d'une culture discontinue de type fed-batch avec commande type PID.

| Symbole | Nom | Valeur | Unité | Intervalle | Source |
|---|---|---|---|---|---|
| $\left(\frac{O_2}{N}\right)_{Sp}$ | Point de consigne $O_2$/N | 25 | $mol_{O2}.mol_N^{-1}$ | [10-120] | Expériences |
| $\left(\frac{O_2}{N}\right)_2$ | Mesure | 23,45641 | $mol_{O2}.mol_N^{-1}$ | [10-120] | Calcul en ligne |
| $\Delta m$ | erreur | 1,23 | $mol_{O2}.mol_N^{-1}$ | [10-120] | Calcul en ligne |
| $r_{CO2}$ | Vitesse de production spécifique de $CO_2$ | 0,44 | $mol_{CO2}.s^{-1}$ | [0-2] | Calcul en ligne |
| SD | Ecart type | 0,15133 | / | [0-1] | Calcul en ligne |
| n | Nombre de mesures | 4 | / | [1-10] | Choisie |
| Cr | Coefficient critique | 0.9 | / | [0.1-2] | Choisie |
| $K_{DB}$ | Coefficient Dead Band | 0.9 | / | [0.1-2] | Choisie |
| P(t) | Facteur proportionnel | 0,02275 | / | [0-1] | Calcul en ligne |
| I(t) | Facteur intégral à t | 0,00076 | / | [0-1] | Calcul en ligne |
| D(t) | Facteur dérivé | 0,00068 | / | [0-1] | Calcul en ligne |
| PID(t) | Valeur de sortie de commande | 0,02419 | / | [1-2] | Calcul en ligne |
| a(t) | Pente du temps de fonctionnement de la pompe de base | 0,2 | $s.s^{-2}$ | [0-1] | Calcul en ligne |

B) Résultats

B1) Essai 1

Afin de valider la commande de type PID visant à minimiser la formation d'acide citrique lors de l'accumulation de lipides par *Yarrowia lipolytica,* deux cultures ont été effectuées. La première a été effectuée sans commande de type PID (alimentation discontinue 1) et la seconde a été effectuée avec le système de commande de type PID (alimentation discontinue 2).

Les trois phases de culture étaient les suivantes :

1. Une croissance exponentielle à $\mu$ = 0,18 $h^{-1}$ et ensuite $\mu$ = $\mu$max imposée par une alimentation limitée en glucose à un pH régulé au moyen d'une solution de $NH_3$.
2. Une diminution de $\mu_{max}$ à $\mu_N$, grâce à une limitation du flux d'azote et un pH régulé au moyen d'une solution d'hydroxyde de potassium.
La croissance des cellules a été réalisée en mode alimentation discontinue l'absence de limitation en oxygène dissous avec une concentration en glucose dans le flacon stock d'alimentation de 740 $g.l^{-1}$.
3. Une phase d'accumulation en présence d'une vitesse de croissance spécifique $\mu$ contrôlée par le flux d'azote et le flux de glucose commandés par l'algorithme de type PID.

➢ Résultats bruts :

Les Figures 2 et 3 représentent les 3 phases de cultures dans les deux cultures : Figure 2 : alimentation discontinue sans contrôleur type PID (alimentation discontinue 1) et Figure 3 : alimentation discontinue avec contrôleur type PID (alimentation discontinue 2)

Les durées des différentes phases étaient comparables dans les deux cultures.

La concentration initiale en biomasse était légèrement supérieure dans la culture à alimentation discontinue avec commande type PID (c'est-à-dire la culture 2). C'est la raison pour laquelle la limitation de l'azote a été effectuée ultérieurement pour l'alimentation discontinue sans commande type PID (c'est-à-dire la culture 1) : une limitation d'azote a été induite à 15,65 h pour la culture 1 et à 9,10 h pour la culture 2, lorsque la concentration en biomasse était proche de 0,5 $Cmol.l^{-1}$.

D'après ces considérations, dans les deux cultures, les profils de concentration en azote étaient similaires, ce qui fait que la limitation d'azote était identique avec et sans commande type PID.

La concentration maximale finale en biomasse était plus élevée lors de la culture à alimentation discontinue 2 en rapport avec une plus faible concentration en acide citrique, comparativement à la culture à alimentation discontinue 1 (voir la figure 7). L'évolution de la concentration en biomasse constante ou même décroissante au cours de la phase d'accumulation de lipides dans la culture 1 a pu être corrélée avec la détérioration des cellules due à la forte concentration en acide citrique (Moeller et coll., (2007), Engineering In Life Sciences). Ces auteurs ont observé que, avec une addition initiale d'acide citrique de 120 $g.l^{-1}$, la productivité d'acide citrique a diminué d'un facteur supérieur à 4, comparativement à une concentration initiale nulle en acide citrique.

Lorsque la limitation d'azote est réalisée, la concentration résiduelle maximale en substrat était plus élevée dans la culture à alimentation discontinue 1 que dans la culture à alimentation discontinue 2, avec respectivement des valeurs de 0,3 et 0,16 $Cmol.l^{-1}$. La concentration résiduelle en substrat était proche de 0 pour la culture à alimentation discontinue 2, avec une valeur de 0 à 0,65 $Cmol.l^{-1}$. Des variations importantes de concentration résiduelle en glucose au cours de la phase d'accumulation de lipides dans la culture 1 ont pu être reliées à un réglage manuel non optimal de la pompe de glucose en fonction du flux d'azote. Ces variations ne se sont pas produites au cours de la culture 2, le réglage de la pompe de glucose s'effectuant via le contrôleur type PID.

• *Concentration en acide citrique*

La Figure 4 représente la variation de la concentration en acide citrique [$Cmol_{citric}.L^{-1}$] au cours du temps dans les cultures avec et sans contrôleur de type PID.

1. au cours de la phase de croissance, les variations de la concentration en acide citrique en fonction du temps étaient très différentes lors de la culture 1 et de la culture 2 : à t = 0, dans la culture 1, la concentration en acide citrique était nulle tandis que, dans la culture 2, la concentration initiale en acide citrique était proche de 0,24 $Cmol.l^{-1}$, Cette faible quantité initiale d'acide citrique a été produite au cours de la préculture.
2. au cours de la phase d'accumulation, pour la culture 1, de 29 h jusqu'à la fin de l'expérience, la concentration en acide citrique a augmenté de 0 à 3,75 $Cmol.l^{-1}$ (voir la figure 6).
3. au cours de la phase d'accumulation, pour la culture 2, la commande de type PID a commencé à la fin de la phase de croissance exponentielle (20 h) et la vitesse spécifique de production d'acide citrique était très faible : la valeur moyenne pour $q_{cit}$ était égale à 0 $\pm$ 0,002 $Cmol_{citrique}.Cmol^{-1}.h^{-1}$ avec des phases alternées de production d'acide citrique et de consommation d'acide citrique en raison du fonctionnement de la commande par rétroaction.

• *Concentration en lipides intracellulaires*

La Figure 5 représente le taux d'accumulation en lipides intracellulaires [$Cmol_{lip}.Cmol_{x]}$ au cours du temps lors des cultures avec et sans contrôleur de type PID.

L'accumulation des lipides est améliorée avec la commande de type PID, avec une teneur totale en lipides supérieure à 0,35 $Cmol_{li}p.Cmolx^{-1}$ tandis que, sans commande PID, la teneur en lipides est proche de 0,25 $Cmol_{lip}.Cmolx^{-1}$ au même âge de culture. En outre, la commande de type PID n'a aucune influence sur le profil en acides gras des lipides accumulés : ceci est mis en évidence sur la figure 6 qui représente le profil des lipides accumulés au cours de cultures en alimentation discontinue (Fed-Batch) de *Yarrowia lipolytica* sur milieu synthétique et glucose, avec commande de type PID durant la phase d'accumulation des lipides : pour chaque composé, les différences de fractions Cmolaires sont inférieures à 7 %.

➢ Paramètres cinétiques

Les paramètres cinétiques des cultures en alimentation discontinue sans et avec commande de type PID (In :

inconnu) présentés dans le tableau suivant ont été calculés d'après les mesures expérimentales.

| | | Sans PID | Avec PID |
|---|---|---|---|
| $\mu_{max}$ | [$h^{-1}$] | 0,258 | 0,257 |
| qs max (croissance) | [$Cmol.Cmol^{-1}.h^{-1}$] | 0,21 | 0,2 |
| qs max (accumulation) | [$Cmol.Cmol^{-1}.h^{-1}$] | 0,11 | 0,09 |
| $q_{lip}$ max | [$Cmol.Cmol^{-1}.h^{-1}$] | 0,036 | 0,044 |
| $q_{citrique}$ | [$Cmol.Cmol^{1}.h^{-1}$] | 0,043 | Un |
| $Y_{X/S}$ | [$Cmol.Cmol^{-1}$] | 0,548 | 0,524 |
| $Y_{lip/S}$ | [$Cmol.Cmol^{-1}$] | 0,103 | 0,372 |
| Ycitrique/S | [$Cmol.Cmol^{-1}$] | 0,323 | In |

D'après le tableau 2, il est clair que les valeurs de $\mu_{max}$, qs $_{max}$ (croissance), $Y_{X/S}$ sont similaires dans les deux cultures. Ces paramètres ne sont pas liés à la production d'acide citrique.

Néanmoins, les valeurs de $q_{lip\ max}$, $Y_{lip/S}$ et $Y_{citrique/S}$ sont très différentes, avec une consommation excessive de substrat pour la synthèse d'acide citrique : le rendement global de conversion du substrat en lipides $Y_{lip/S}$ est environ 3 fois plus faible dans la culture 1, que dans la culture 2.

La Figure 7 représente le taux de production de lipides spécifiques au cours de culture avec commande type PID. Les variations du signal supérieures à 50% apparaissent au démarrage de la phase sous commande type PID (20 h ; 26 h). L'apparition d'oscillations met en évidence des valeurs de la commande type PID inappropriées. La réponse corrective est trop forte en comparaison d'une action de correction optimale. La production et la consommation d'acide apparaissent alternativement. Les paramètres sont alors ajustés afin d'améliorer la stabilité du système de contrôle (27 h) et la déviation standard du signal PID décroit dans la gamme [10% - 15%]

L'accumulation des lipides est favorisée en présence d'une faible vitesse de croissance spécifique [0,02 ; 0,05 $h^{-1}$]. A ces valeurs de $\mu$, la demande de glucose est réduite même si la concentration en biomasse est supérieure à 50 $g.l^{-1}$, avec un débit du glucose d'environ 0,0004 $Cmol.h^{-1}$. Il est donc évident que la commande de la pompe devait être précise.

Aucune consommation de lipides n'est observée.

Lorsque $QR_{consommation\ de\ lipides}$ < QR $_{production\ d'acide\ citrique}$, l'algorithme type PID a ajusté le débit du glucose lorsque la levure a commencé à consommer les lipides accumulés.

La vitesse spécifique de production de lipides a atteint dans ces travaux des valeurs élevées de 0,04 $Cmol_{lipides}.Cmolx^{-1}.h^{-1}$, tandis que la valeur $q_{citrique}$ est nulle. Une valeur de sortie du PID(t) adaptée a permis une augmentation de la valeur de $q_{lipides}$ (27 h - 57 h). A 72 h, le point de consigne de PID(t) a été ajusté à 35 [u] de telle sorte que la valeur de $q_{lipides}$ augmente de 0,025 à 0,044 $Cmol_{lipides}.Cmolx^{-1}.h^{-1}$ en 10 h. Cette observation a révélé une valeur optimale de point de consigne plus près de 35 que de 25 [u].

Note : les désignations de signal PID(t) au cours de la période [50-70 h] doivent être mises en rapport avec la différence entre le temps de réponse biologique de la levure et le temps de réponse physique de la pompe de substrat. Les dynamiques physiques sont différentes des dynamiques biologiques.

➢ Paramètres de commande

La Figure 8 représente les variations du coefficient respiratoire (QR) et du rapport $\frac{r_C}{r_N}$ par rapport au temps au cours de culture sans commande PID.

La Figure 9 représente les variations du coefficient respiratoire (QR) et du rapport $\frac{r_C}{r_N}$ par rapport au temps au cours de culture avec commande PID.

D'après les évolutions de QR, au cours de la phase de croissance, pour les deux cultures, la valeur de QR est pratiquement constante, proche de 1. Au cours de la phase d'accumulation de lipides, la valeur de QR diminue jusqu'à 0,7 pour la culture à alimentation discontinue 1 et augmente lentement jusqu'à 1,19 pour la culture 2.

D'après les évolutions de $\frac{r_C}{r_N}$ au cours des deux cultures, en phase de croissance (phase I), de faibles

variations de $\frac{r_C}{r_N}$ sont observées.

Au cours de la culture à alimentation discontinue 2, $\frac{r_C}{r_N}$ augmente rapidement jusqu'à 14 (Cmol.volume de réacteu$^{-1}$.h$^{-1}$).(Nmol.volume de réacteur$^{-1}$.h$^{-1}$)$^{-1}$ puis devient stable, avec une valeur de 13 à 15 (Cmol.volume de réacteur$^{-1}$.h$^{-1}$).(Nmol.volume de réacteur$^{-1}$.h$^{-1}$)$^{-1}$.

Au cours de la culture à alimentation discontinue 1, le ratio $\frac{r_C}{r_N}$ n'est pas contrôlé ni régulé et il varie en fonction du réglage manuel de débit de la pompe de substrat. Ce rapport de flux non contrôlé entre le carbone et l'azote conduit à la production d'une grande quantité d'acide citrique dans la culture 1, avec une valeur de RQ inférieure à 1.

La Figure 10 représente les variations temporelles des flux de la pompe d'alimentation en substrat [$Cmol_S$.$Cmol_X^{-1}$.L$^{-1}$] et du volume de composé basique ajouté au cours de cultures avec ou sans commande de type PID.

En tenant compte de la différence en concentration initiale de la biomasse, les variations de débit de la pompe d'alimentation en substrat sont identiques dans les deux cultures au cours des phases de croissance.

Au cours de la phase d'accumulation de lipides,

- de la culture 1, le débit de la pompe d'alimentation en substrat est manuellement ajusté en fonction de $\mu$ et du flux d'azote afin de limiter la production d'acide citrique.
- pour la culture 2, le débit de la pompe d'alimentation en substrat est contrôlé et régulé grâce au système de type PID. Le débit de la pompe croit lentement en fonction de l'augmentation de concentration en biomasse.

Concernant le volume de composé basique ajouté au cours de la phase de croissance, les quantités sont similaires dans les deux cultures (0,1 mol.$Cmol_X^{-1}$ de l'ammoniac pour la culture 1 et 0,12 mol. $Cmol_X^{-1}$ pour la culture 2). Pour la phase d'accumulation, le volume de composé basique ajouté diffère de façon importante entre les cultures 1 et 2, avec des valeurs respectives de 3710 mL et 200 mL.

L'ajout important de base dans la culture 1 est nécessaire pour neutraliser l'accumulation d'acide citrique dans le milieu de culture.

B2) Essai 2

Un autre essai a été effectué avec du glycérol comme source de carbone.

La stratégie de culture détaillée dans le texte ci dessus a été mise en oeuvre avec le contrôleur de type PID selon une stratégie de co-substrat : glucose et glycérol. L'apport en glycérol est réalisé lors de la phase d'accumulation de lipides et à partir de 40 h de culture en réacteur, après que le métabolisme d'accumulation lipidique à partir de glucose ait débuté. Le débit relatif de glycérol (défini par le ratio du débit de glycérol sur le débit total de glucose et glycérol) est augmenté par pallier jusqu'à un flux 100% glycérol (voir Figure 12)

Le contrôleur est utilisé lorsque le glycérol est l'unique substrat en phase d'accumulation de lipides. Les valeurs du rapport entre la vitesse de consommation de carbone et la vitesse de consommation de l'azote ($r_C/r_N$) et le rapport entre la vitesse de consommation d'oxygène et la vitesse de consommation d'azote ($r_{O2}/r_N$) sont respectivement comprises entre 12,6 et 14,4, (mole de carbone consommé par mole d'azote consommé) et entre 22,7 et 25,9 (mole d'oxygène consommé par mole de d'azote consommé).

Une quantité de biomasse totale de 60 Cmol est produite avec une concentration finale de 5,87 Cmol.L$^{-1}$ (120 g.L$^{-1}$) en 110 heures. La teneur en lipide maximale atteinte est de 0,48 $Cmol_{lip}$.$Cmol_X^{-1}$. (Figure 13).

La concentration en acide citrique maximale atteinte est de 0,8 mCmol.L$^{-1}$ alors que la concentration maximale en glycérol est inférieure à 1 mCmol.L$^{-1}$ (Figure14).

Les résultats expérimentaux de la culture sont résumés dans le Tableau ci-dessous présentant les résultats des cultures de *Y. lipolytica* sauvage à 28 °C, à pH 5,5 avec le glucose comme unique substrat et en double substrat glucose/glycérol.

| Variables | Unité | Glycérol sans PID | Glycérol avec PID |
|---|---|---|---|
| [acide citrique]$_{max}$ | [$Cmol_{citr}$.L$^{-1}$] | 0,23 | 0,01 |
| $q^*_{lip\ max}$ | [$Cmol_{lip}$.$Cmol_x^{-1}$,h$^{-1}$] | 0,025 | 0,031 |
| $R_{S/X^*max}$ | [$Cmol_X$.$Cmol_{glc}^{-1}$] | 0,56 | 0,57 |

(suite)

| Variables | Unité | Glycérol sans PID | Glycérol avec PID |
|---|---|---|---|
| $R_{S/lip\ max}$ | $[Cmol_{lip}.Cmol_{glc}^{-1}]$ | 0,13 | 0,20 |

En comparant les performances obtenues sans et avec le contrôleur des apports de substrats, il apparaît que lors des deux cultures, le même rendement apparent de conversion du glycérol en biomasse est identique et égal à 0,57 $[Cmol_X.Cmol_{glc}^{-1}]$.

Ce tableau met en évidence que

- l'accumulation lipidique avec le glycérol comme unique substrat pendant la phase d'accumulation est favorisée par la stratégie définie dans le présent texte et l'utilisation du contrôleur : le rendement de conversion du glycérol en lipides est égal à 0,20 $Cmol_{lip}.Cmol_{gly}^{-1}$, valeur supérieure à celle issue de la culture réalisée sans contrôle des apports en substrats et valeur supérieure à celle déterminée par Papanikolaou en culture continue (0,19 $Cmol_{lip}.Cmol_{gly}^{-1}$) (S. PAPANIKOLAOU & G. AGGELIS (2002) Lipid production by *Yarrowia lipolytica* growing on industrial glycerol in a single-stage continuous culture. Bioresource Technol., 82(1), 43-492002)) ;
- la vitesse spécifique de production de lipides sous contrôle des paramètres opératoires comme précisé dans le présent texte est supérieure de 20 % par rapport à la culture sans contrôle ;
- la stratégie de culture et l'utilisation du contrôleur définies dans le présent texte permet de minimiser la production d'acide citrique dans le milieu de culture.

Ainsi, il est démontré que la mise en oeuvre de la stratégie et du mode de contrôle/commande définis selon l'invention permet de minimiser la production d'acide citrique tout en maximisant l'accumulation de lipides par *Y. lipolytica* que ce soit avec le glucose ou le glycérol comme seul source de carbone.

Seul compte l'ajustement des paramètres qui doit être préalablement établi.

B3) Tests PID

La robustesse de l'algorithme est prouvée lors des d'essais réalisés sous différentes conditions perturbantes (agitation, ajout d'antimousse, aération...).

Les résultats sont présentés sur la Figure 11 avec les variations du signal PID et du débit de glucose en fonction du temps.

Après chaque prélèvement, le système est perturbé: il est observé un pic de 100% $\pm$ 7% du signal PID(t) pendant 13 min $\pm$ 1,2 min après l'échantillonnage.

Ces événements illustrent la capacité de l'algorithme à ajuster le débit de la pompe de glucose lors d'une diminution soudaine de la quantité de biomasse dans le bioréacteur.

L'algorithme prend en compte la variation de la quantité de biomasse liée à l'échantillonnage et diminue le flux de glucose afin d'éviter l'apparition d'un excès de carbone.

La durée de la diminution du débit de la pompe de glucose est calculée en fonction de la vitesse de croissance de la levure. L'ajustement du débit de la pompe permet d'adapter le flux de glucose à la biomasse présente dans le système.

Pendant une phase transitoire, le débit est sub-optimal mais il revient rapidement à une consigne optimale.

C) Conclusion

Dans ces travaux, une stratégie de commande initiale a été élaborée pour optimiser les performances de *Yarrowia lipolytica* en termes du rendement de conversion du glucose en lipides, de la productivité et de la capacité de mise en réserve des lipides. Ce mode de commande, similaire à un algorithme PID, était basé sur le calcul de $\frac{r_{O_2}}{r_N}$ et sur le temps de fonctionnement de la pompe de base d'après les mesures de capteurs en ligne. Il a été possible de commander le débit de la pompe de glucose pour optimiser l'accumulation des lipides sans production d'acide citrique et d'obtenir les plus hautes performances indiquées dans la littérature : 0,020 $Cmol.Cmol^{-1}.h^{-1}$. La fiabilité et la reproductibilité de l'algorithme ont été démontrées ; pour toutes les cultures effectuées avec le même mode de commande au moyen de différentes souches de *Yarrowia lipolytica*, la production d'acide citrique a été réduite au minimum et la concentration en acide citrique était pratiquement nulle.

## Revendications

1. Procédé de culture par alimentation discontinue de levures du genre *Yarrowia,* particulièrement de la souche *Yarrowia lipolytica,* **caractérisé en ce que** le milieu de culture comprend outre les nutriments essentiels nécessaires à la croissance de levures du genre *Yarrowia,* du glucose seul et que la valeur du rapport entre la vitesse de consommation de carbone et la vitesse de consommation d'azote ($r_C/r_N$) est contrôlée et fixée à une valeur définie, pour favoriser la production de lipides et limiter la production d'acide citrique lorsque la culture est en phase d'accumulation de lipides.

2. Procédé selon la revendication 1, **caractérisé en ce que**

   - le rapport entre la vitesse de consommation de carbone et la vitesse de consommation de l'azote ($r_C/r_N$) et le coefficient respiratoire (QR) ; ou bien
   - le rapport entre la vitesse de consommation de carbone et la vitesse de consommation de l'azote ($r_C/r_N$) et le rapport entre la vitesse de consommation d'oxygène et la vitesse de consommation d'azote ($r_{O2}/r_N$) ou
   - le rapport entre la vitesse de consommation d'oxygène et la vitesse de consommation d'azote ($r_{O2}/r_N$) et QR (rapport entre la vitesse de production de dioxyde de carbone ($r_{co2}$) et la vitesse de consommation d'oxygène ($r_{O2}$)),

   avantageusement le rapport entre la vitesse de consommation de carbone et la vitesse de consommation de l'azote ($r_C/r_N$) et et le rapport ($r_{O2}/r_N$), sont contrôlés et fixés à des valeurs définies, pour optimiser la production de lipides et limiter la production d'acide citrique.

3. Procédé selon l'une quelconque des revendications 1 ou 2, **caractérisé en ce que** le rapport entre la vitesse de consommation de carbone et la vitesse de consommation de l'azote ($r_C/r_N$) est maintenu à une valeur comprise entre 12 et 16, préférentiellement entre 13 et 15 (mole de carbone consommé par mole d'azote consommé).

4. Procédé selon l'une quelconque des revendications 2 ou 3, **caractérisé en ce que** la valeur du coefficient respiratoire (QR) est maintenue entre 0,9 et 1,30 préférentiellement entre 1,01 et 1,11 (mole de $CO_2$ produite par mole de d'oxygène consommée).

5. Procédé selon l'une quelconque des revendications 2 à 4, **caractérisé en ce que** la valeur du rapport entre la vitesse de consommation d'oxygène et la vitesse de consommation d'azote ($r_{O2}/r_N$) peut être maintenue entre 21 et 27 préférentiellement entre 22 et 26 (mole d'oxygène consommée par mole de d'azote consommé).

6. Procédé selon l'une quelconque des revendications 1 à 5, **caractérisé en ce que**

   - le rapport entre la vitesse de consommation de carbone et la vitesse de consommation de l'azote ($r_C/r_N$) ;
   - le rapport entre la vitesse de consommation de carbone et la vitesse de consommation de l'azote ($r_C/r_N$) et le coefficient respiratoire (QR) ; ou bien
   - le rapport entre la vitesse de consommation de carbone et la vitesse de consommation de l'azote ($r_C/r_N$) et le rapport entre la vitesse de consommation d'oxygène et la vitesse de consommation d'azote ($r_{O2}/r_N$) ou
   - le rapport entre la vitesse de consommation d'oxygène et la vitesse de consommation d'azote ($r_{O2}/r_N$) et QR ;

   sont contrôlés et fixés à des valeurs définies de manière automatisée.

7. Procédé selon la revendication 6, **caractérisé en ce que** l'automatisation est réalisée au travers d'un contrôleur de type PID.

8. Procédé selon la revendication 7, **caractérisé en ce que** le contrôleur de type PID intervient sur le débit glucose introduit dans le fermenteur dans lequel la culture de levures du genre *Yarrowia,* particulièrement de la souche *Yarrowia lipolytica* est réalisée.

9. Procédé selon l'une quelconque des revendications 7 ou 8, **caractérisé en ce que** le contrôleur de type PID intervient sur le débit nominal de la pompe d'alimentation en glucose (PID(t)) et de l'apport de source azotée.

10. Procédé selon la revendication 8, **caractérisé en ce que** le contrôleur PID(t) répond à l'algorithme

$$PID(t) = \Delta m * K_p * r_{CO_2} + (I(t - 1) + \Delta m) * K_i * r_{CO_2} + a(t) * r_{CO_2} * K_d$$

dans lequel

- $\Delta$m représente l'écart entre la valeur de $\frac{r_{O_2}}{r_N}$ mesurée et la valeur de la consigne ;
- $K_P$ représente le facteur proportionnel (c'est un facteur correctif ajustable par l'utilisateur) ;
- $r_{CO2}$ représente la vitesse de production dioxyde de carbone considérée pour le volume total du système étudié soit le volume réactionnel total du réacteur, comprenant la biomasse, les phases liquide et gaz ;
- I(t - 1) représente l'action intégrale au temps t moins une unité de temps ;
- $K_i$ représente le facteur intégral (c'est un facteur correctif ajustable par l'utilisateur ;
- a(t) représente la pente de la courbe (temps de fonctionnement de la pompe d'alimentation en composé basique) = f(t) dans une fenêtre de temps glissante ;
- $K_d$ représente le facteur dérivé (facteur correctif ajustable par l'utilisateur).

**11.** Dispositif de culture de *Yarrowia lipolytica* comprenant au moins un fermenteur muni des moyens d'alimentation en éléments nécessaires à la culture de levures du genre *Yarrowia,* particulièrement de la souche *Yarrowia lipolytica* et d'un moyen de contrôle de la valeur du rapport entre la vitesse de consommation de carbone et la vitesse de consommation de l'azote ($r_C/r_N$), ou du rapport entre la vitesse de consommation de carbone et la vitesse de consommation de l'azote ($r_C/r_N$) et le coefficient respiratoire (QR) ou du rapport entre la vitesse de consommation de carbone et la vitesse de consommation de l'azote ($r_C/r_N$) et le rapport entre la vitesse de consommation d'oxygène et la vitesse de consommation d'azote ($r_{O2}/r_N$) ou du rapport entre la vitesse de consommation d'oxygène et la vitesse de consommation d'azote ($r_{O2}/r_N$) et QR, **caractérisé en ce que** le moyen de maintenir aux valeurs fixées du rapport entre la vitesse de consommation de carbone et la vitesse de consommation de l'azote ($r_C/r_N$), ou du rapport entre la vitesse de consommation de carbone et la vitesse de consommation de l'azote ($r_C/r_N$) et le coefficient respiratoire (QR) ou du rapport entre la vitesse de consommation de carbone et la vitesse de consommation de l'azote ($r_C/r_N$) et le rapport entre la vitesse de consommation d'oxygène et la vitesse de consommation d'azote ($r_{O2}/r_N$) ou du rapport entre la vitesse de consommation d'oxygène et la vitesse de consommation d'azote ($r_{O2}/r_N$) et QR est un contrôleur de type PID tel que décrit à l'une quelconque des revendications 7 à 10.

## Patentansprüche

**1.** Verfahren zur Feed-Batch-Kultivierung von Hefen der Gattung *Yarrowia,* insbesondere des Stamms *Yarrowia lipolytica,*
**dadurch gekennzeichnet, dass** das Kulturmedium neben den essentiellen Nährstoffen, die für das Wachstum von Hefen der Gattung *Yarrowia* notwendig sind, nur Glucose umfasst, und dass der Wert des Verhältnisses zwischen der Geschwindigkeit des Verbrauchs von Kohlenstoff und der Geschwindigkeit des Verbrauchs von Stickstoff ($r_C/r_N$) gesteuert und auf einen definierten Wert festgelegt wird, um die Erzeugung von Lipiden zu fördern und die Erzeugung von Zitronensäure zu begrenzen, wenn sich die Kultur in der Phase zur Akkumulation von Lipiden befindet.

**2.** Verfahren nach Anspruch 1,
**dadurch gekennzeichnet, dass**:

- das Verhältnis zwischen der Geschwindigkeit des Verbrauchs von Kohlenstoff und der Geschwindigkeit des Verbrauchs von Stickstoff ($r_C/r_N$) und der Atmungskoeffizient (QR); oder auch
- das Verhältnis zwischen der Geschwindigkeit des Verbrauchs von Kohlenstoff und der Geschwindigkeit des Verbrauchs von Stickstoff ($r_C/r_N$) und das Verhältnis zwischen der Geschwindigkeit des Verbrauchs von Sauerstoff und der Geschwindigkeit des Verbrauchs von Stickstoff ($r_{O2}/r_N$) oder
- das Verhältnis zwischen der Geschwindigkeit des Verbrauchs von Sauerstoff und der Geschwindigkeit des Verbrauchs von Stickstoff ($r_{O2}/r_N$) und QR (Verhältnis zwischen der Geschwindigkeit der Erzeugung von Kohlendioxid ($r_{CO2}$) und der Geschwindigkeit des Verbrauchs von Sauerstoff ($r_{O2}$)),

vorteilhaft das Verhältnis zwischen der Geschwindigkeit des Verbrauchs von Kohlenstoff und der Geschwindigkeit des Verbrauchs von Stickstoff ($r_C/r_N$) und das Verhältnis ($r_{O2}/r_N$), gesteuert und auf definierte Werte festgelegt

werden, um die Erzeugung von Lipiden zu optimieren und die Erzeugung von Zitronensäure zu begrenzen.

**3.** Verfahren nach einem der Ansprüche 1 oder 2,
**dadurch gekennzeichnet, dass** das Verhältnis zwischen der Geschwindigkeit des Verbrauchs von Kohlenstoff und der Geschwindigkeit des Verbrauchs von Stickstoff ($r_C/r_N$) auf einem Wert zwischen 12 und 16, vorzugsweise zwischen 13 und 15 (Mol verbrauchter Kohlenstoff durch Mol verbrauchter Stickstoff), gehalten wird.

**4.** Verfahren nach einem der Ansprüche 2 oder 3,
**dadurch gekennzeichnet, dass** der Wert des Atmungskoeffizienten (QR) zwischen 0,9 und 1,30, vorzugsweise zwischen 1,01 und 1,11 (Mol erzeugtes $CO_2$ durch Mol verbrauchter Sauerstoff), gehalten wird.

**5.** Verfahren nach einem der Ansprüche 2 bis 4,
**dadurch gekennzeichnet, dass** der Wert des Verhältnisses zwischen der Geschwindigkeit des Verbrauchs von Sauerstoff und der Geschwindigkeit des Verbrauchs von Stickstoff ($r_{O2}/r_N$) zwischen 21 und 27, vorzugsweise zwischen 22 und 26 (Mol verbrauchter Sauerstoff durch Mol verbrauchter Stickstoff), gehalten werden kann.

**6.** Verfahren nach einem der Ansprüche 1 bis 5, **dadurch gekennzeichnet, dass**:

- das Verhältnis zwischen der Geschwindigkeit des Verbrauchs von Kohlenstoff und der Geschwindigkeit des Verbrauchs von Stickstoff ($r_C/r_N$) ;
- das Verhältnis zwischen der Geschwindigkeit des Verbrauchs von Kohlenstoff und der Geschwindigkeit des Verbrauchs von Stickstoff ($r_C/r_N$) und der Atmungskoeffizient (QR); oder auch
- das Verhältnis zwischen der Geschwindigkeit des Verbrauchs von Kohlenstoff und der Geschwindigkeit des Verbrauchs von Stickstoff ($r_C/r_N$) und das Verhältnis zwischen der Geschwindigkeit des Verbrauchs von Sauerstoff und der Geschwindigkeit des Verbrauchs von Stickstoff ($r_{O2}/r_N$) oder
- das Verhältnis zwischen der Geschwindigkeit des Verbrauchs von Sauerstoff und der Geschwindigkeit des Verbrauchs von Stickstoff ($r_{O2}/r_N$) und QR, automatisiert gesteuert und auf definierte Werte festgelegt werden.

**7.** Verfahren nach Anspruch 6,
**dadurch gekennzeichnet, dass** die Automatisierung durch eine Steuereinheit vom PID-Typ vorgenommen wird.

**8.** Verfahren nach Anspruch 7,
**dadurch gekennzeichnet, dass** die Steuereinheit vom PID-Typ den Durchsatz von Glucose regelt, die in den Fermenter eingebracht wird, in dem die Kultivierung von Hefen der Gattung *Yarrowia,* insbesondere des Stamms *Yarrowia lipolytica,* vorgenommen wird.

**9.** Verfahren nach einem der Ansprüche 7 oder 8,
**dadurch gekennzeichnet, dass** die Steuereinheit vom PID-Typ den nominellen Durchsatz der Förderpumpe von Glucose (PID(t)) und der Zufuhr der Stickstoffquelle regelt.

**10.** Verfahren nach Anspruch 8,
**dadurch gekennzeichnet, dass** die Steuereinheit PID(t) dem Algorithmus

$$\mathrm{PID}(t) = \Delta m * K_p * r_{CO_2} + (I(t-1) + \Delta m) * K_i * r_{CO_2} + a(t) * r_{CO_2} * K_d$$

folgt, wobei

- $\Delta m$ die Abweichung zwischen dem gemessenen Wert von 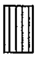 und dem Sollwert repräsentiert;
- $K_P$ den Proportionalfaktor repräsentiert (der ein vom Benutzer einstellbarer Korrekturfaktor ist);
- $r_{CO2}$ die Geschwindigkeit der Erzeugung von Kohlendioxid bezogen auf das Gesamtvolumen des untersuchten Systems repräsentiert, d.h. das gesamte Reaktionsvolumen des Reaktors, umfassend die Biomasse, die Flüssig- und Gasphase;
- I(t-1) die Integralfunktion zur Zeit 1 minus einer Zeiteinheit repräsentiert;
- $K_i$ den Integralfaktor repräsentiert (der ein vom Benutzer einstellbarer Korrekturfaktor ist);
- a(t) die Steigung der Kurve repräsentiert (Betriebszeit der Förderpumpe der Basiszusammensetzung) = f(t) in einem gleitenden Zeitfenster;

- $K_d$ den Ableitungsfaktor repräsentiert (vom Benutzer einstellbarer Korrekturfaktor).

**11.** Vorrichtung zur Kultivierung von *Yarrowia lipolytica,* umfassend mindestens einen Fermenter, versehen mit Mitteln zur Zufuhr von Elementen, die für die Kultivierung von Hefen der Gattung *Yarrowia,* insbesondere des Stamms *Yarrowia lipolytica,* notwendig sind, und mit einem Mittel zur Steuerung des Werts des Verhältnisses zwischen der Geschwindigkeit des Verbrauchs von Kohlenstoff und der Geschwindigkeit des Verbrauchs von Stickstoff ($r_C/r_N$), oder des Verhältnisses zwischen der Geschwindigkeit des Verbrauchs von Kohlenstoff und der Geschwindigkeit des Verbrauchs von Stickstoff ($r_C/r_N$) und des Atmungskoeffizienten (QR), oder des Verhältnisses zwischen der Geschwindigkeit des Verbrauchs von Kohlenstoff und der Geschwindigkeit des Verbrauchs von Stickstoff ($r_C/r_N$) und des Verhältnisses zwischen der Geschwindigkeit des Verbrauchs von Sauerstoff und der Geschwindigkeit des Verbrauchs von Stickstoff ($r_{O2}/r_N$) oder des Verhältnisses zwischen der Geschwindigkeit des Verbrauchs von Sauerstoff und der Geschwindigkeit des Verbrauchs von Stickstoff ($r_{O2}/r_N$) und von QR,
**dadurch gekennzeichnet, dass** das Mittel zur Aufrechterhaltung auf Festwerten des Verhältnisses zwischen der Geschwindigkeit des Verbrauchs von Kohlenstoff und der Geschwindigkeit des Verbrauchs von Stickstoff ($r_C/r_N$), oder des Verhältnisses zwischen der Geschwindigkeit des Verbrauchs von Kohlenstoff und der Geschwindigkeit des Verbrauchs von Stickstoff ($r_C/r_N$) und des Atmungskoeffizienten (QR), oder des Verhältnisses zwischen der Geschwindigkeit des Verbrauchs von Kohlenstoff und der Geschwindigkeit des Verbrauchs von Stickstoff ($r_C/r_N$) und des Verhältnisses zwischen der Geschwindigkeit des Verbrauchs von Sauerstoff und der Geschwindigkeit des Verbrauchs von Stickstoff ($r_{O2}/r_N$) oder des Verhältnisses zwischen der Geschwindigkeit des Verbrauchs von Sauerstoff und der Geschwindigkeit des Verbrauchs von Stickstoff ($r_{O2}/r_N$) und von QR eine Steuereinheit vom PID-Typ ist, wie in einem der Ansprüche 7 bis 10 beschrieben.

## Claims

**1.** Method for the batch-fed culture of yeast of the genus *Yarrowia,* particularly of the strain *Yarrowia lipolytica,* **characterized in that** the culture medium comprises, aside from the essential nutrients necessary for the growth of yeast of the genus *Yarrowia,* glucose alone, and **in that** the value of the ratio between the carbon consumption rate and the nitrogen consumption rate ($r_C/r_N$) is controlled and fixed at a defined value, to promote the production of lipids and limit the production of citric acid when the culture is in the lipid accumulation phase.

**2.** Method according to claim 1, **characterized in that**

- the ratio between the carbon consumption rate and the nitrogen consumption rate ($r_C/r_N$) and the respiratory quotient (RQ); or else
- the ratio between the carbon consumption rate and the nitrogen consumption rate ($r_C/r_N$) and the ratio between the oxygen consumption rate and the nitrogen consumption rate ($r_{O2}/r_N$), or
- the ratio between the oxygen consumption rate and the nitrogen consumption rate ($r_{O2}/r_N$) and RQ (ratio between the carbon dioxide production rate ($r_{CO2}$) and the oxygen consumption rate ($r_{O2}$)),

advantageously the ratio between the carbon consumption rate and the nitrogen consumption rate ($r_C/r_N$) and the ratio ($r_{O2}/r_N$), are controlled and fixed at defined values, to optimize the production of lipids and limit the production of citric acid.

**3.** Method according to either one of claims 1 and 2, **characterized in that** the ratio between the carbon consumption rate and the nitrogen consumption rate ($r_C/r_N$) is kept at a value of between 12 and 16, preferentially between 13 and 15 (moles of carbon consumed per mole of nitrogen consumed).

**4.** Method according to either one of claims 2 and 3, **characterized in that** the value of the respiratory quotient (RQ) is kept between 0.9 and 1.30, preferentially between 1.01 and 1.11 (moles of $CO_2$ produced per mole of oxygen consumed).

**5.** Method according to any one of claims 2 to 4, **characterized in that** the value of the ratio between the oxygen consumption rate and the nitrogen consumption rate ($r_{O2}/r_N$) may be kept between 21 and 27, preferentially between 22 and 26 (moles of oxygen consumed per mole of nitrogen consumed).

**6.** Method according to any one of claims 1 to 5, **characterized in that**

- the ratio between the carbon consumption rate and the nitrogen consumption rate ($r_C/r_N$);
- the ratio between the carbon consumption rate and the nitrogen consumption rate ($r_C/r_N$) and the respiratory quotient (RQ); or else
- the ratio between the carbon consumption rate and the nitrogen consumption rate ($r_C/r_N$) and the ratio between the oxygen consumption rate and the nitrogen consumption rate ($r_{O2}/r_N$), or
- the ratio between the oxygen consumption rate and the nitrogen consumption rate ($r_{O2}/r_N$) and RQ;

are controlled and fixed at defined values in an automated manner.

**7.** Method according to claim 6, **characterized in that** the automation is carried out via a controller of PID type.

**8.** Method according to claim 7, **characterized in that** the controller of PID type acts on the flow rate of glucose introduced into the fermenter in which the yeast of genus *Yarrowia,* particularly of the strain *Yarrowia lipolytica,* is being cultured.

**9.** Method according to either one of claims 7 and 8, **characterized in that** the controller of PID type acts on the nominal flow rate of the glucose supply pump (PID(t)) and of the supply of the nitrogenous source.

**10.** Method according to claim 8, **characterized in that** the controller PID(t) responds to the algorithm

$$PID(t) = \Delta m * K_p * r_{CO_2} + (I(t-1) + \Delta m) * K_I * r_{CO_2} + a(t) * r_{CO_2} * K_d$$

in which

- $\Delta$m represents the difference between the measured value of 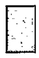 and the setpoint value,
- $K_P$ represents the proportional factor (which is a corrective factor which may be adjusted by the user);
- $r_{CO2}$ represents the carbon dioxide production rate considered for the total volume of the system studied, in other words the total reaction volume of the reactor, comprising the biomass and the liquid and gas phases;
- I(t-1) represents the integral action at the time t, minus one unit of time;
- $K_i$ represents the integral factor (which is a corrective factor which may be adjusted by the user);
- a(t) represents the slope of the curve (operating time of the basic compound supply pump) = f(t) in a sliding window of time;
- $K_d$ represents the derivative factor (corrective factor which may be adjusted by the user).

**11.** Device for culturing *Yarrowia lipolytica* comprising at least one fermenter provided with means for supplying the elements necessary for the culture of yeast of the genus *Yarrowia,* particularly of the strain *Yarrowia lipolytica,* and a means for controlling the value of the ratio between the carbon consumption rate and the nitrogen consumption rate ($r_C/r_N$), or the ratio between the carbon consumption rate and the nitrogen consumption rate ($r_C/r_N$) and the respiratory quotient (RQ), or the ratio between the carbon consumption rate and the nitrogen consumption rate ($r_C/r_N$) and the ratio between the oxygen consumption rate and the nitrogen consumption rate ($r_{O2}/r_N$), or the ratio between the oxygen consumption rate and the nitrogen consumption rate ($r_{O2}/r_N$) and RQ, **characterized in that** the means for keeping the ratio between the carbon consumption rate and the nitrogen consumption rate ($r_C/r_N$), or the ratio between the carbon consumption rate and the nitrogen consumption rate ($r_C/r_N$) and the respiratory quotient (RQ), or the ratio between the carbon consumption rate and the nitrogen consumption rate ($r_C/r_N$) and the ratio between the oxygen consumption rate and the nitrogen consumption rate ($r_{O2}/r_N$), or the ratio between the oxygen consumption rate and the nitrogen consumption rate ($r_{O2}/r_N$) and RQ at the fixed values is a controller of PID type as described in any one of claims 7 to 10.

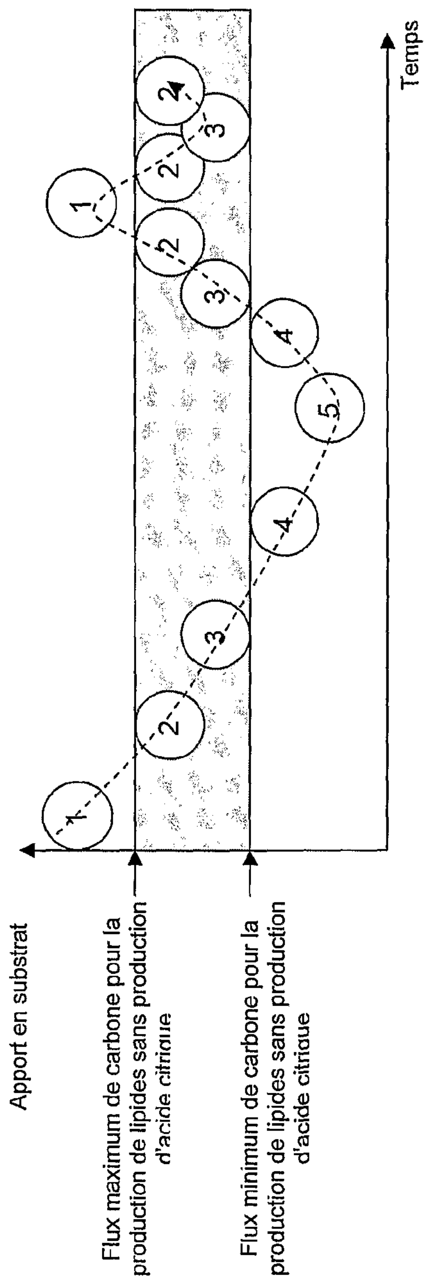
Apport en substrat
Flux maximum de carbone pour la production de lipides sans production d'acide citrique
Flux minimum de carbone pour la production de lipides sans production d'acide citrique
Temps

FIGURE 1

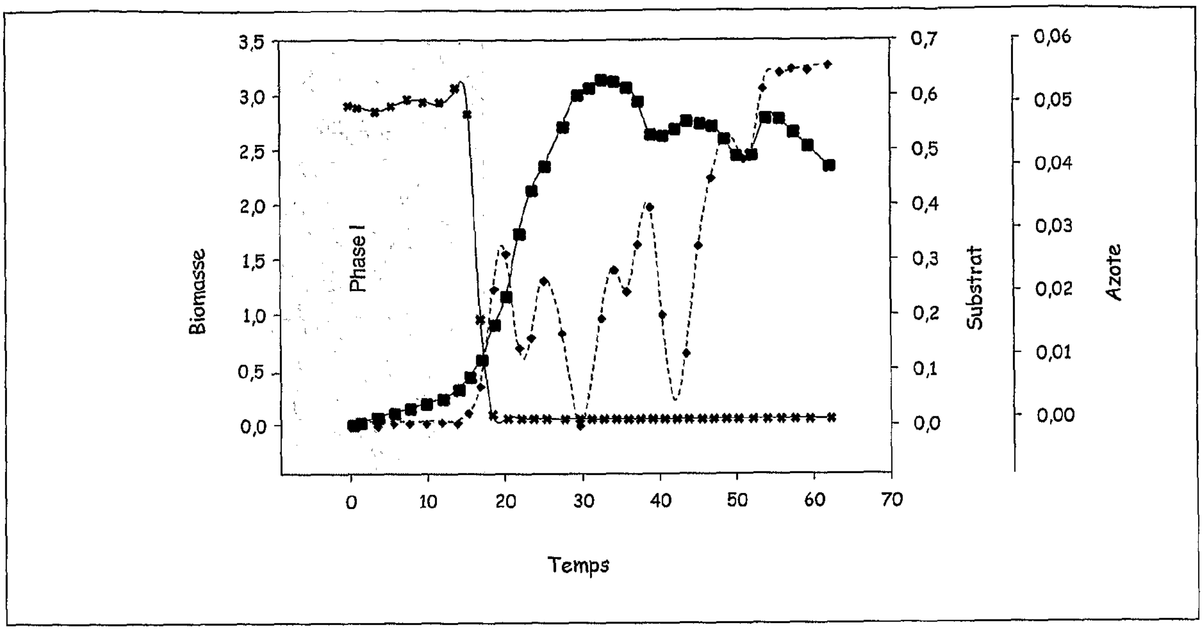


FIGURE 2

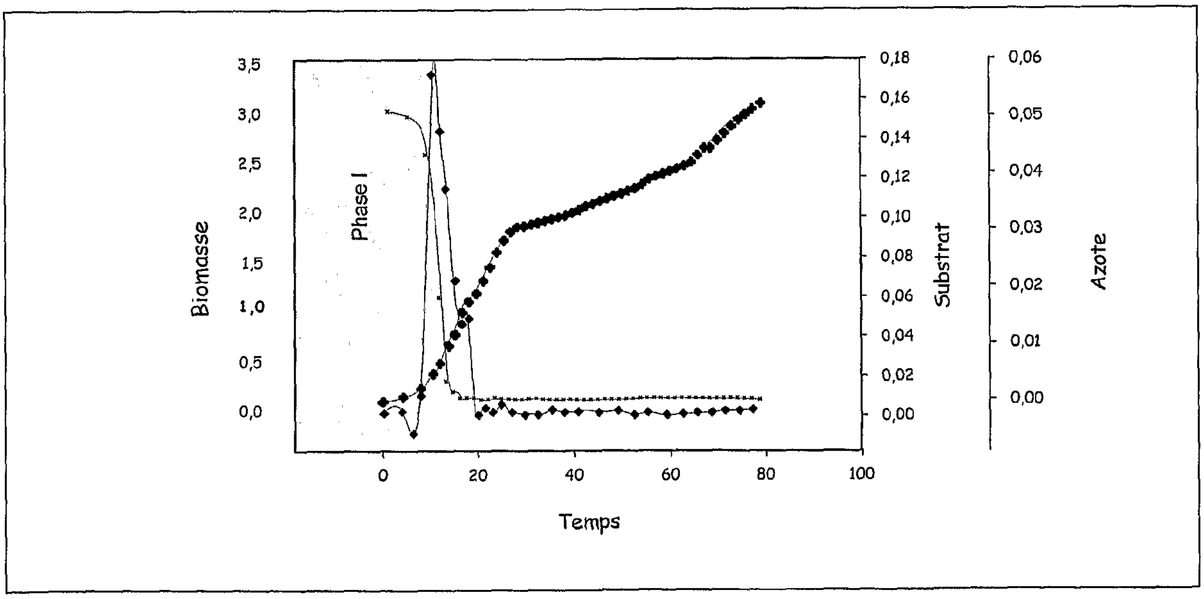


FIGURE 3

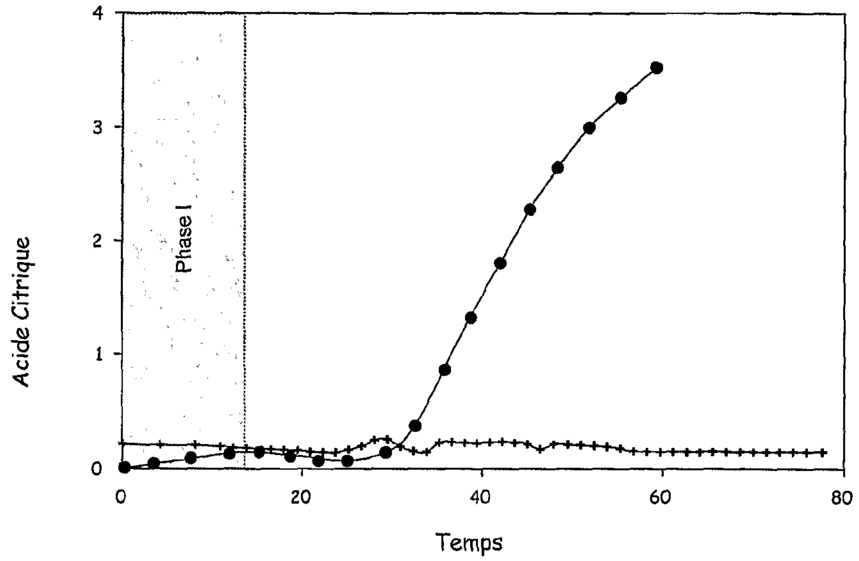


FIGURE 4

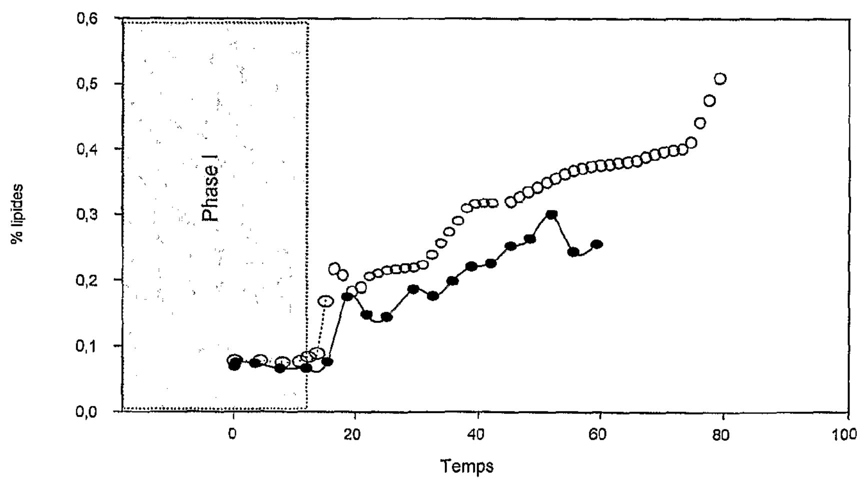


FIGURE 5

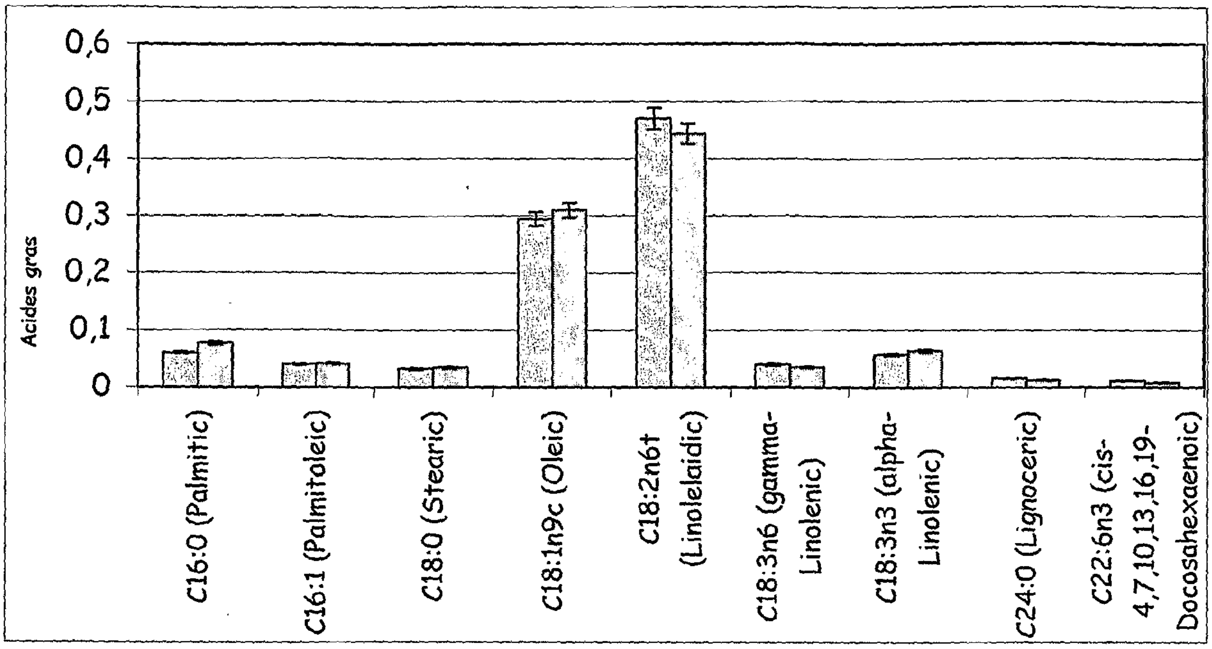


FIGURE 6

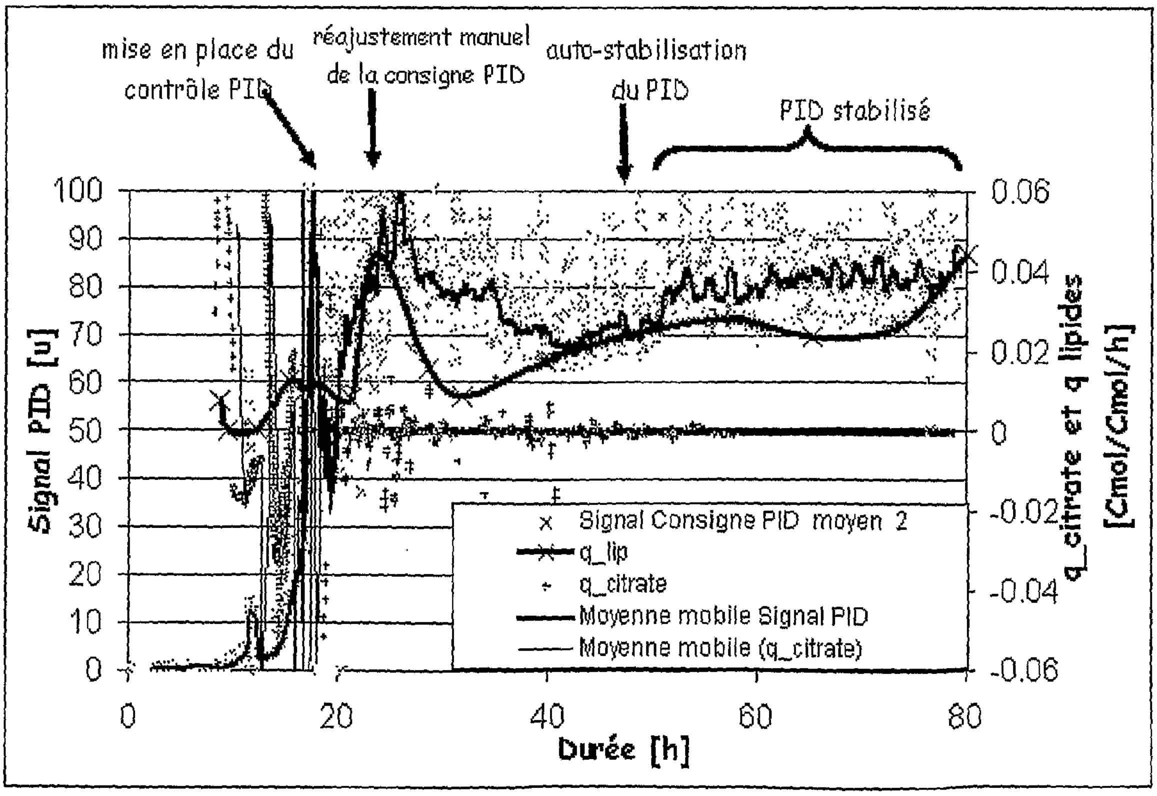


FIGURE 7

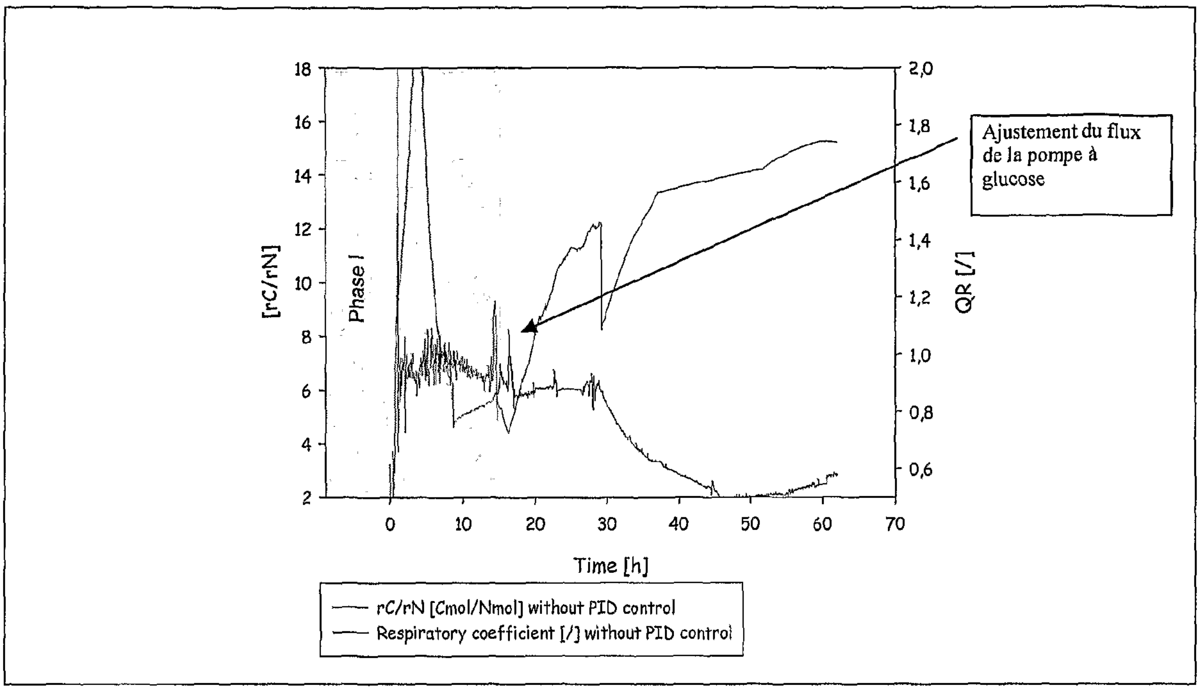


FIGURE 8

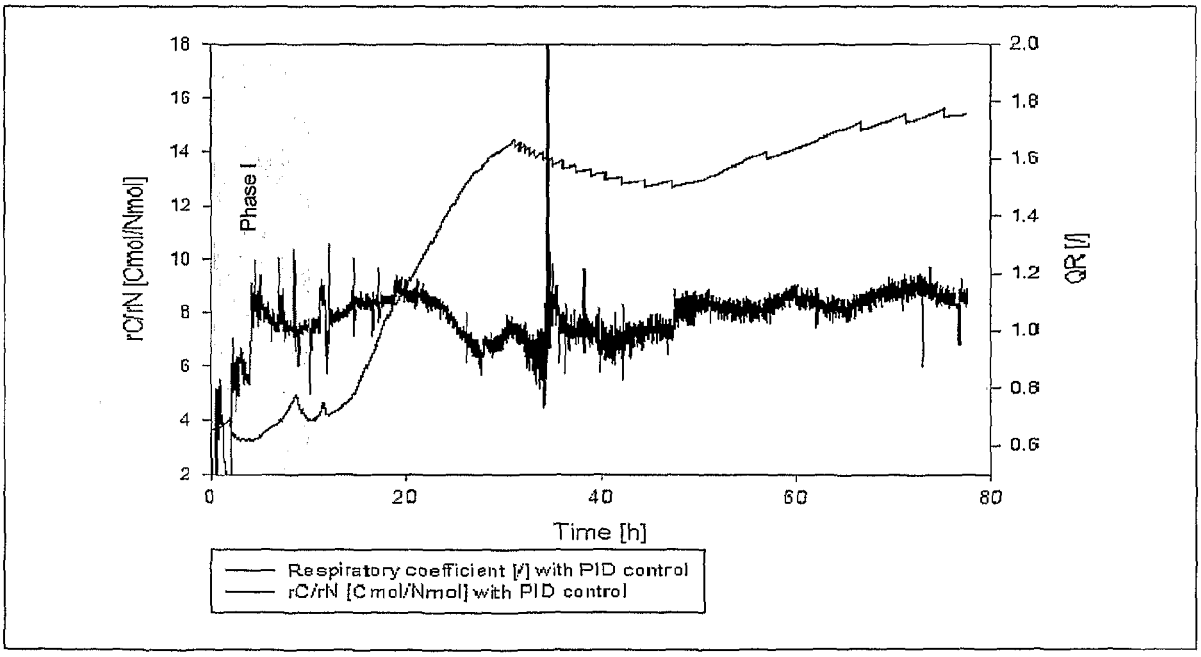


FIGURE 9

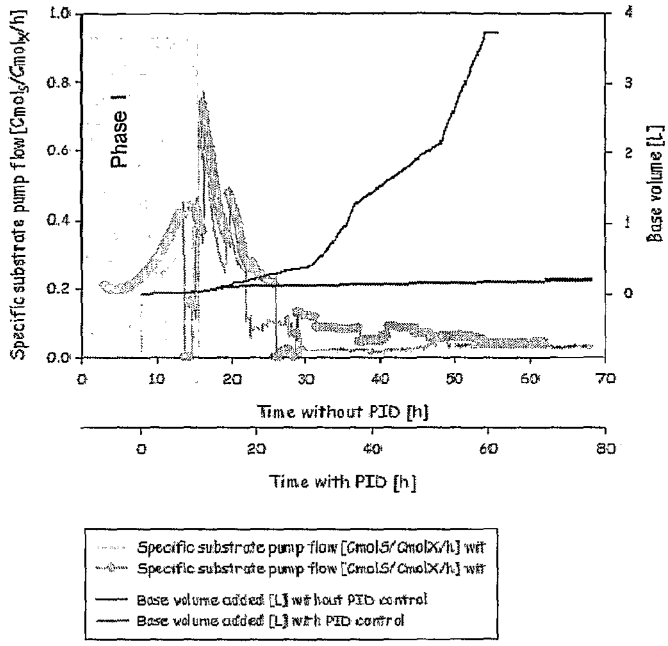


FIGURE 10

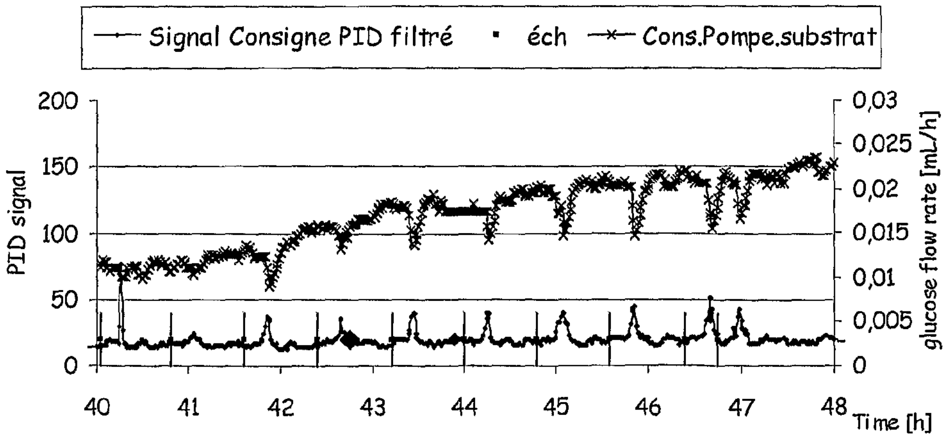


FIGURE 11

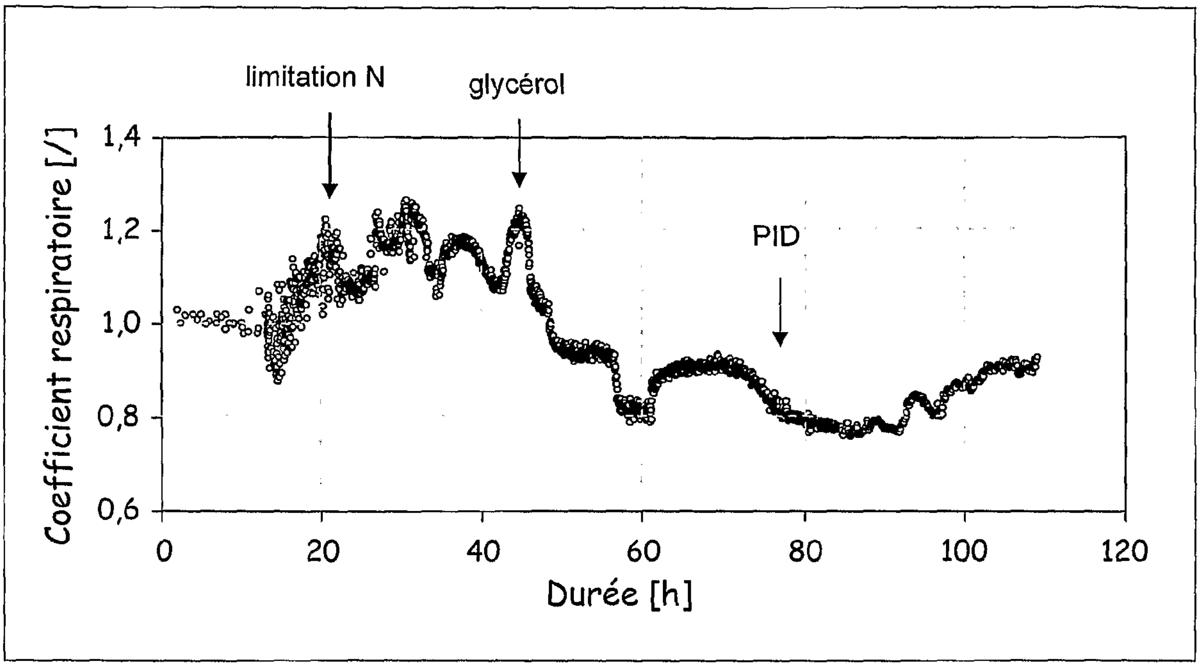


FIGURE 12

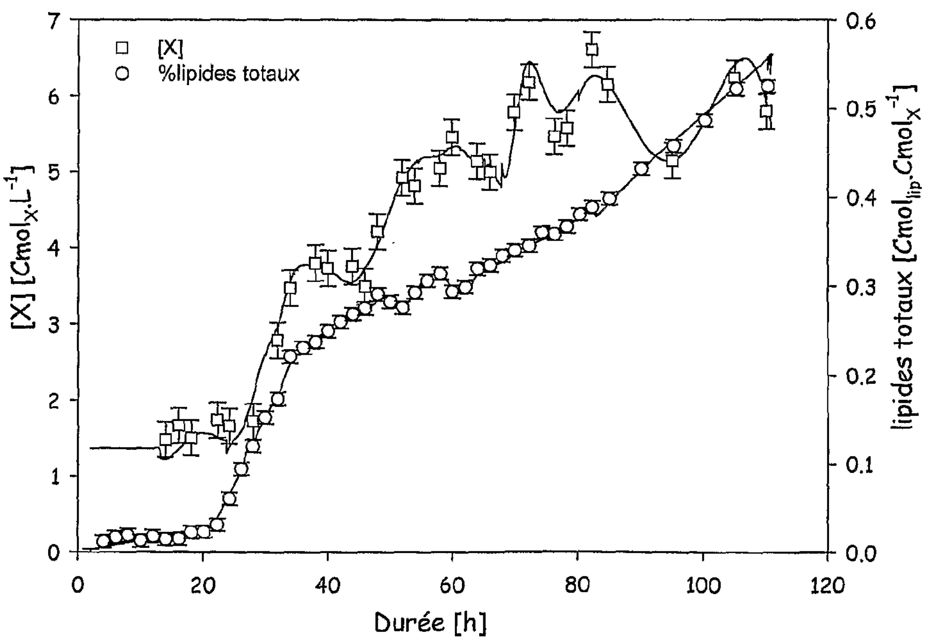


FIGURE 13

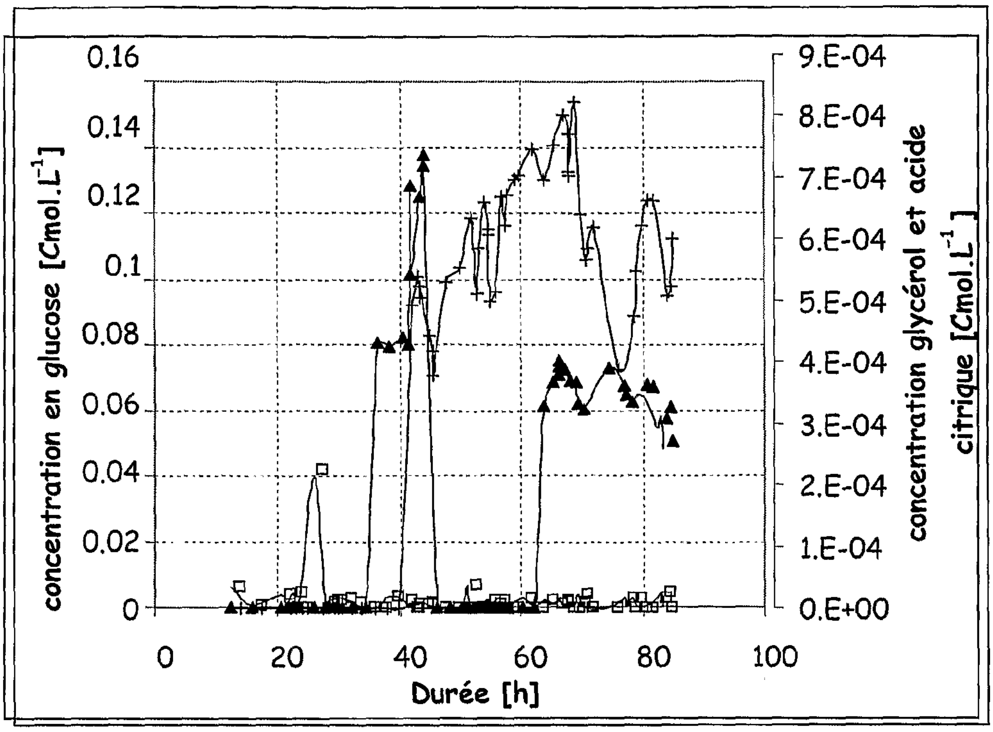
0.16
0.14
0.12
0.1
0.08
0.06
0.04
0.02
0
concentration en glucose [Cmol.L-1]
9.E-04
8.E-04
7.E-04
6.E-04
5.E-04
4.E-04
3.E-04
2.E-04
1.E-04
0.E+00
concentration glycérol et acide citrique [Cmol.L-1]
0
20
40
60
80
100
Durée [h]

FIGURE 14

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **ARNDT, M.** *Computers & Chemical Engineering,* 2005, vol. 29, 1113
- **HITZMANN, B.** *Bioprocess and Biosystems Engineering,* 2000, vol. 23, 337
- **VICENTE, A.** *Biotechnology and Bioengineering,* 1997, vol. 58, 445-450
- **HUTH, J.** *J. Basic Microbiol.,* vol. 30 (7), 481-488
- *J. BASIC MICROBIOL,* vol. 30 (7), 489-497
- *J. BASIC MICROBIOL,* vol. 30 (8), 561-567
- **ROOS, W.** *J. Gen. Microbiol.,* 1984, vol. 130, 1007-1014
- **ISHIZAKI, A.** *J. Ferment. Bioeng,* 1994, vol. 77, 541-547
- **IVERSEN, J.J.L.** *Appl. Microbiol. Biotechnol.,* 1994, vol. 42, 256-262
- **SAN, K.-Y. ; STEPHANOPOULOS, G.** Biotechnol. Bioeng. 1984, vol. 26, 1209-1218
- **SHIOYA, S.** *Elsevier Applied Science,* 1989, 15-22
- **SIANO, S.A.** *Biotechnol. Bioeng.,* 1995, vol. 47, 651-665
- *Bioresource Technol.,* vol. 82 (1), 43-492002